# EUROPEAN PATENT SPECIFICATION

(11) **EP 1 417 629 B1**
(45) Date of publication and mention of the grant of the patent: **20.02.2008**
(21) Application number: 02749828.6
(22) Date of filing: 05.07.2002
(51) Int. Cl.: G06G 7/48, G01N 33/68, G01N 33/92

(54) **GENERATING, VIEWING, INTERPRETING, AND UTILIZING A QUANTITATIVE DATABASE OF METABOLITES**
ERZEUGEN, BETRACHTEN, INTERPRETIEREN UND VERWENDEN EINER QUANTITATIVEN DATENBANK VON METABOLITEN
METHODES PERMETTANT DE GENERER, VISUALISER, INTERPRETER ET UTILISER UNE BASE DE DONNEES QUANTITATIVES DE METABOLITES

(30) Priority: 06.07.2001 US 303704 P
(43) Date of publication of application: 12.05.2004
(73) Proprietor: Lipomics Technologies, Inc., West Sacramento, CA 95691 (US)
(72) Inventor: WATKINS, Steven, M., Sacramento, CA 95835-1606 (US)
(74) Representative: Hoarton, Lloyd Douglas Charles
(86) International application number: PCT/US2002/021426
(87) International publication number: WO 2003/005628

(56) References cited:
- US-A- 5 952 235
- US-B2- 6 576 471
- STANTON B ET AL: "Interaction of estrogen and 2,3,7,8-tetrachlorodibenzo-p-dioxin (TCDD) with hepatic fatty acid synthesis and metabolism of male chickens (Gallus domesticus)." COMPARATIVE BIOCHEMISTRY AND PHYSIOLOGY. TOXICOLOGY & PHARMACOLOGY : CBP. JUN 2001, vol. 129, no. 2, June 2001 (2001-06), pages 137-150, XP002332804 ISSN: 1532-0456
- WATKINS S M ET AL: "Unique phospholipid metabolism in mouse heart in response to dietary docosahexaenoic or alpha-linolenic acids" LIPIDS, vol. 36, no. 3, March 2001 (2001-03), pages 247-254, XP008048857 ISSN: 0024-4201
- BERNHARDT T G ET AL: "Purification of fatty acid ethyl esters by solid-phase extraction and high-performance liquid chromatography" JOURNAL OF CHROMATOGRAPHY B : BIOMEDICAL APPLICATIONS, ELSEVIER SCIENCE PUBLISHERS, NL, vol. 675, no. 2, 26 January 1996 (1996-01-26), pages 189-196, XP004043412 ISSN: 0378-4347
- FIEHN O ET AL: "COMIBNING GENOMICS, METABOLOME ANALYSIS, AND BIOCHEMICAL MODELLING TO UNDERSTAND METABOLIC NETWORKS" COMPARATIVE AND FUNCTIONAL GENOMICS, vol. 2, no. 3, June 2001 (2001-06), pages 155-168, XP008014995 ISSN: 1531-6912
- DATABASE BIOSIS [Online] WATKINS ET AL.: 'Individual metabolism should guide agriculture toward foods for improved health and nutrition', XP002967118 Retrieved from STN Database accession no. 2001:448083 & AMERICAN J. CLINICAL NUTRITION vol. 74, no. 3, September 2001, pages 283 - 286
- DATABASE BIOSIS [Online] SAUDEK ET AL.: 'Lipid metabolism in diabetes mellitus', XP002967119 Retrieved from STN Database accession no. 1979:257631 & AMERICAN JOURNAL OF MEDICINE vol. 66, no. 5, 1979, pages 843 - 853
- DATABASE BIOSIS [Online] WATERS ET AL.: 'NMR and pattern recognition studies on liver extracts and intact livers from rats treated with alpha-naphthylisothiocyanate', XP002967120 Retrieved from STN Database accession no. 2002:462434 & BIOCHEMICAL PHARMACOLOGY vol. 64, no. 1, July 2002, pages 67 - 77
- DATABASE BIOSIS [Online] JARDINE ET AL.: 'Fluvastatin in combination with cyclosporing in renal transplant recipients', XP002967121 Retrieved from STN Database accession no. 2000:119959 & JOURNAL OF CLINICAL PHARMACY AND THERAPEUTICS vol. 24, no. 6, December 1999, pages 397 - 408
- DATABASE BIOSIS [Online] LITHELL H.: 'Hypertension and hyperlipidemia: a review', XP002967122 Retrieved from STN Database accession no. 1994:57024
- JARDINE A.; HOLDAAS A.: J. CLINICAL PHARMACY AND THERAPEUTICS, vol. 24, 1999, pages 397-408,

## Description

### FIELD

This disclosure relates to ways of quantifying metabolites and collecting quantitative data on metabolites, a database of quantified metabolite profiles, and methods of mining and visualizing selected subsets thereof.

### BACKGROUND

The recent explosion of data acquisition and analysis technology, termed informatics, promises to revolutionize predictive and diagnostic medicine. The information readily available to doctors and scientists today dwarfs that of even a few years ago, and will expand at an even more accelerated rate in the next few years. Managing this information and applying it to useful purpose are formidable challenges.

Currently, genomics is the most developed and recognized form of biological informatics. Genomics developed to simultaneously identify the elements of heredity and to assign biological function to these elements. Despite the inherent complexity of the genome, the invention of just a few molecular tools enabled genomics to flourish into the science known today. In the near future, it is likely that most common genetic diseases will have been identified, many using genomic tools. The power of the knowledge emerging from the genome is that identifying the genetic basis of an inherited disease can provide logical strategies to treat those afflicted on an individual basis. However, genomics is not a panacea for predictive medicine because phenotype is not necessarily predicted by genotype. Beyond its application to diseases with demonstrably genetic causes, however, the direct utility of genomics by itself diminishes.

Ultimately, changes in phenotype and not changes in genes (genotype) are of direct interest to nutrition and health. The gap between genotype and phenotype is spanned by many biochemical steps, each with individual specificities and a sensitivity to various influences, including diet and the environment In the chain of biomolecules from genes to phenotype, metabolites are the quantifiable molecules with the closest link to phenotype. Many phenotypic and genotypic states are characterized or predicted by differences in the concentration of metabolites within biological tissues or fluids. For example, the progression of coronary artery disease can be predicted by the serum concentration of cholesterol and the presence of non-insulin dependant diabetes is characterized by elevated plasma free fatty acids.

Metabolite informatics, or metabolomics, represents a more logical approach than genomics for identifying trends or metabolic profiles of specific diseases. While the assessment of disease in man has been pursued using individual metabolite assessments, there are no technologies that enable the accumulation of diverse metabolome data in a single seamless and expandable resource. Such a resource would allow global metabolic effects of disparate affectors to be compared and contrasted. Data for such a resource would need to be quantitative so that data from many investigators, analytical, technologies, and sample matrices could be integrated and compared. A quantitative database of metabolites containing samples from systems treated with many affectors or expressing many phenotypic or genotypic traits could be used to identify the molecular mechanisms consistent and divergent across many biological systems and individual samples and sample collections.

Early attempts to use a metabolomic strategy for investigating phenotype have proven valuable across a broad spectrum of biological research. In microbiology, changes in metabolite profiles were used to describe the global metabolic response and variable glucose metabolism of *E. coli* under different growth conditions (Tweeddale et al., J. Bacteriology 180:5109-5116, 1998), Metabolome analyses were also used to identify the global changes in *E. coli* metabolism caused by changes in population density (Liu et al., J. Bacteriology 182:4158-4164, 2000). Raamsdonk et al. (Nature Biotechnology 19:45-50, 2001) used metabolomic analyses of yeast to identify the metabolic function of deleted genes for which there was no observable phenotypic consequence of their deletion. Using metabolomics to identify the function of genes demonstrates the versatility and power of metabolomics. Unlike genomics and proteomics, metabolomics can be used to identify changes that occur at all levels of biology from genes to environment. The direct results of nutritional; genomic or expression differences can be observed in a metabolite profile. This strategy is also widely accepted in plant research as a method for screening for desirable traits, and for understanding the phenotypic expression of genes (Fiehn et al., Nature Biotechnology 18:1157-1161, 2000; Glassbrook et al., Nature Biotecnology 18:1157-1161, 2000). Watkins et al, Lipids, Vol. 36, No. 3, 2001, discloses a study of the effect of dietary docosahexaenoic and alpha-linolenic acids on phospholipid metabolism in mouse hearts. The fatty acid composition of lipid classes from the hearts was analyzed. Internal standards were used in this analysis. Stanton et al, Comparative Biochemistry and Physiology, Part C, 129, 137-150 (2001), discloses a study of the effect of estrogen and TCDD on hepatic fatty acid synthesis and metabolism of male chickens. Internal standards were used in the analysis of the hepatic lipids.

What is needed is a system for creating a quantitative bioinformatic database of metabolites, such as lipid metabolites, suitable for integrative research and valid comparative studies across many disciplines and sample systems. Further, there is a need to develop easy, understandable tools for mining, visualizing and interpreting this bioinformatic resource. Technologies are needed that can create and interact with accessible annotated databases of metabolite concentrations reflective of individuals in various phenotypic states.

### SUMMARY OF THE INVENTION

This invention provides methods for analyzing a plurality of quantitative lipid metabolite profiles as defined by the appended claims.

### BRIEF DESCRIPTION OF THE FIGURES

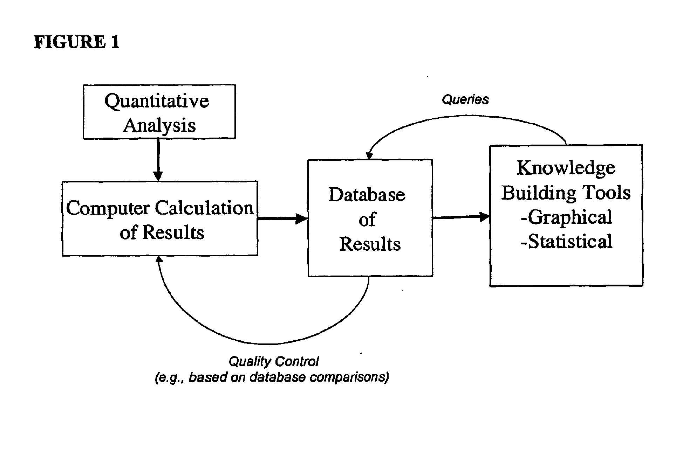

**Figure 1** is a schematic overview of metabolomic analysis as provided herein. Quantitative analysis is used to measure a plurality of metabolites from a sample; the raw data produced by such analysis is optionally subjected to one or more transformations (e.g., computer calculations), including for instance integration of the area under a chromatogram curve with or without correction. Raw data and/or transformed data are entered into a database of results. In certain of the provided embodiments, a quality control mechanism compares the entered data against existing data in the database and identifies aberrant or erroneous data, which may lead to re-testing or repeated analysis. The database can be queried, for instance using filters or other discrimination mechanisms, and subsets of data that fit the query displayed.

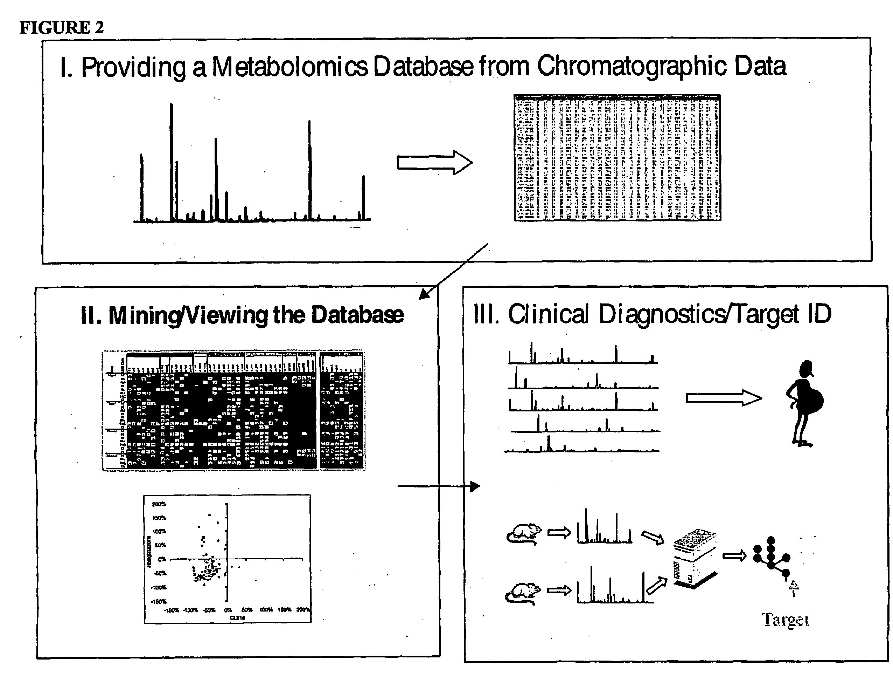

**Figure 2** is a schematic representation of certain lipid metabolite analysis embodiments. Chromatographic data is entered into a database, which can be mined for desired information and presented in the form of a graphical interface of a a heat map. Such an interface may be optionally provided in interactive form on a computer system, or remotely across, for instance, the Internet or another computer communication system. Data mined from the cumulative lipid metabolite database can be used, for instance, for clinical or diagnostic testing (*e*.*g*., for a propensity to obesity), or to identify specific metabolic targets of drugs, as described in more detail herein.

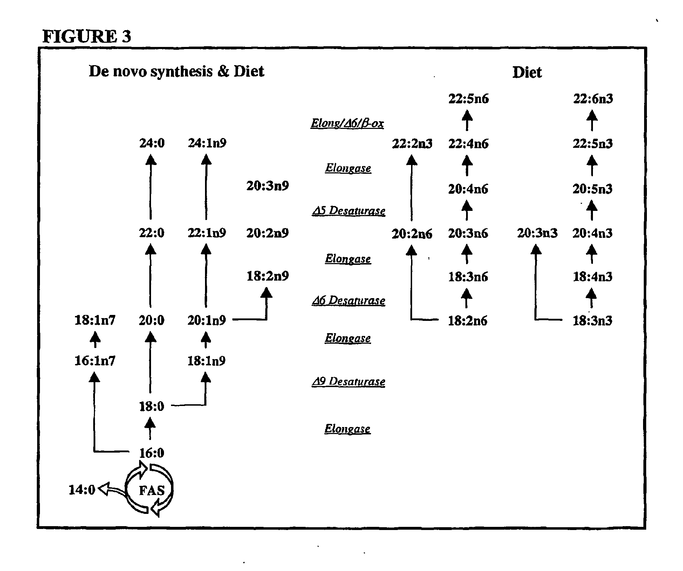

**Figure 3** is a diagram showing an overview of the pathways of de novo fatty acid metabolism in humans.

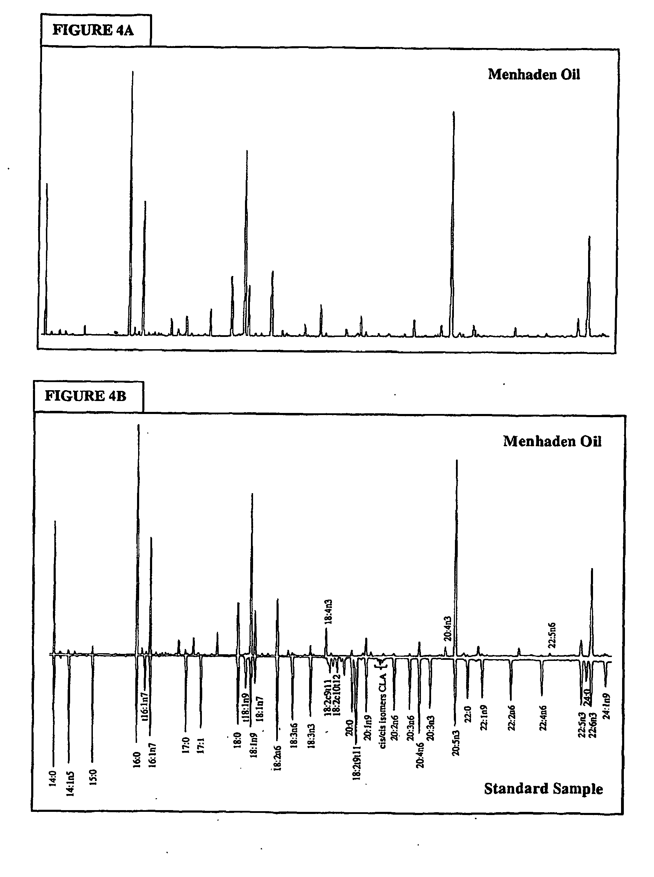

**Figure 4** is a set of chromatograms of the indicated samples, produced by gas chromatography. FIG 4A shows the gas chromatogram of a sample of Menhaden oil. FIG 4B shows a mirrored chromatogram, in which the Menhaden oil chromatogram is displayed top to bottom with a control chromatogram that contains standard compounds for comparison (labeled "Standard Sample"). Major peaks are identified as indicated.

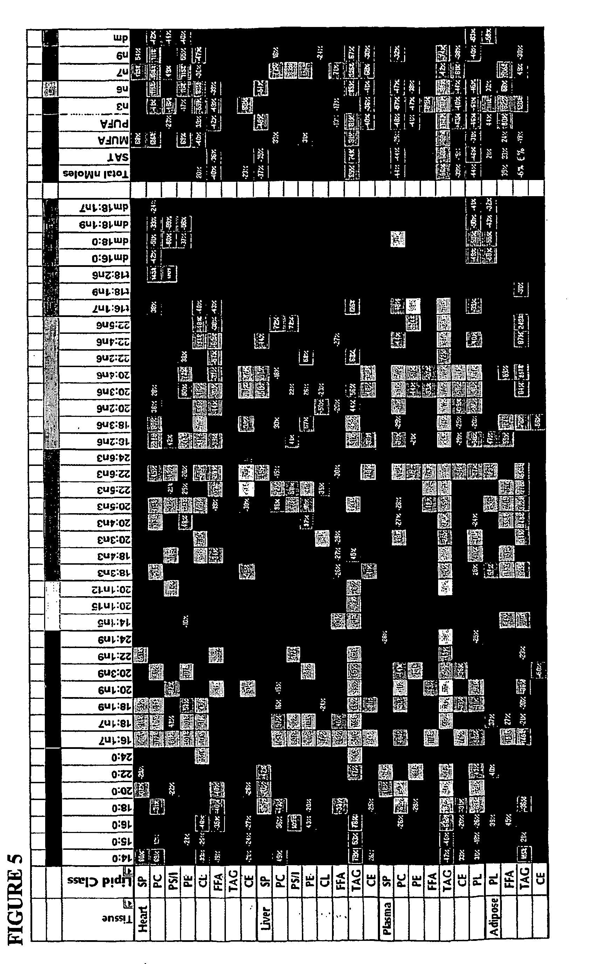

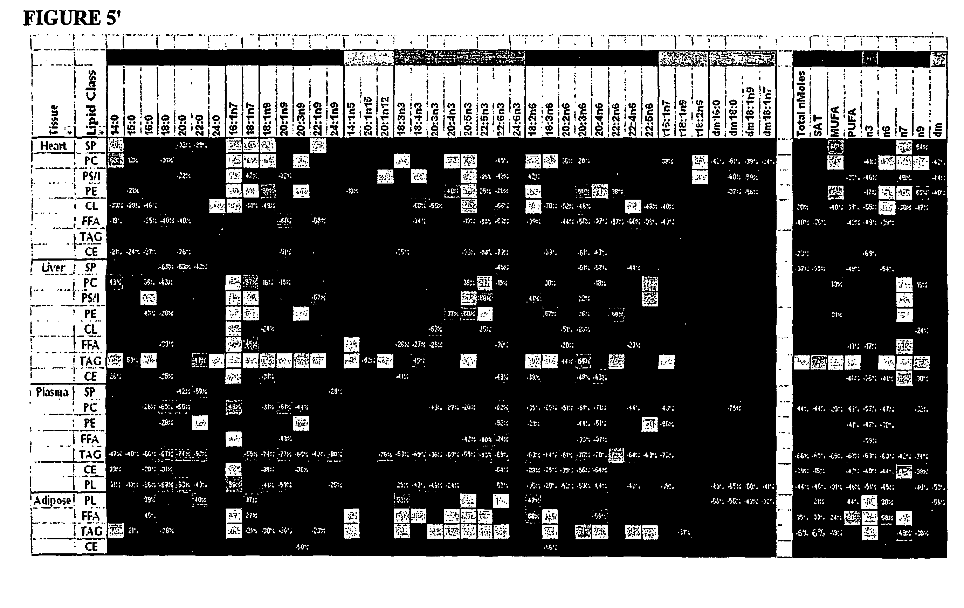

**Figure 5** is a representative "heat map" display of lipomic data, illustrating effects of rosiglitazone treatment on individual lipid metabolites. The concentration (expressed in nmol/g sample) of each lipid metabolite from treated and untreated mice was used to generate a heat map. The tissue and lipid class of each sample is indicated in the row headers (left). The fatty acid or sterol is indicated in the column headers (top). Color coding indicates the percentage difference between a control sample and the test sample, as explained below and in Example 1. The column headers represent an individual fatty acid present in the lipid classes, which are displayed on the left. The magnitude of the difference, expressed as a percentage change in the quantitative data between treated and untreated mice, is represented by color according to the legend. Differences not meeting a P < 0.05 are displayed in black.

Summary data is presented in the smaller chart to the right, and includes nM of each fatty acid for each tissue: (1) total fatty acids, (2) saturated fatty acids, (3) mono-unsaturated fatty acids, (4) poly-unsaturated fatty acids, (5) n3, (6) n6, (7) n7, (8) n9 unsaturated fatty acids, and (9) plasmalogens ("dm").

**Figure 5****'** is a duplicate of Figure 5, but is printed in grey-tones rather than in color.

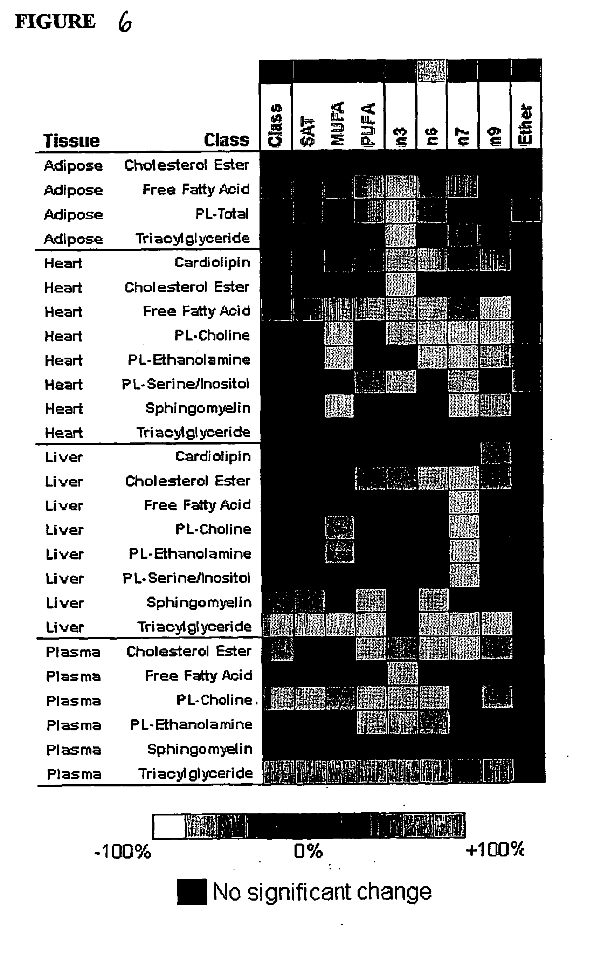

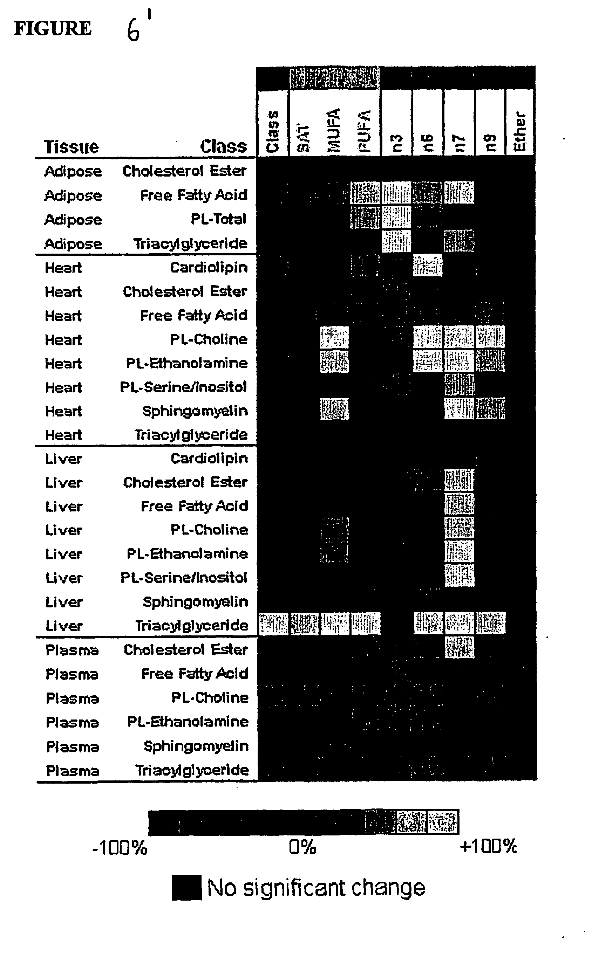

**Figure 6** shows an example of a heat map indicating that rosiglitazone treatment exerts strong and tissue-specific effects on lipid class metabolism. The concentration (expressed in nmol/g sample) of each lipid metabolite from treated and untreated mice was used to generate the summary data displayed here as a heat map. The first column displays the quantitative difference in the concentration of each lipid class between the groups. The next columns, in order, describe the quantitative difference in the concentration of saturated fatty acids, monounsaturated fatty acids, polyunsaturated fatty acids, n3 fatty acids, n6 fatty acids, n7 fatty acids, n9 fatty acids, and plasmalogen lipids among the groups. The magnitude of the difference, expressed as a percentage change in the quantitative data between treated and untreated mice, is represented by color.

**Figure 6****'** is a duplicate of **Figure 6**, but is printed in grey-tones rather than in color.

### DETAILED DESCRIPTION

### I. Abbreviations

| | |
|---|---|
| **CDP-DAG:** | CDP-diacylglycerol |
| **CE:** | cholesterol ester |
| **CL:** | cardiolipin |
| **DAG:** | diacylglycerides |
| **FAME:** | fatty acid methyl ester |
| **FFA:** | free fatty acid |
| **LMP:** | lipid metabolite profile |
| **LY:** | lyso-phosphatidylcholine |
| **LyCL:** | lysocardiolipin |
| **LyPE:** | lysophosphatidylethanolamine |
| **MAG:** | monoacylglycerides |
| **PA:** | phosphatidic acid |
| **PC:** | phosphotidylcholine |
| **PE:** | phosphatidylethanolamine |
| **PG:** | phosphatidylglycerol |
| **PI:** | phosphotidylinositol |
| **PS:** | phosphotidylserine |
| **PS/I:** | phosphotidylinositol / phosphotidylserine |
| **SP:** | sphingomyelin |
| **TAG:** | triacylglycerol |

### II. Explanation of Certain Terms

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments, the following explanations of certain terms are provided:

Biological Sample: Any biological material, such as a cell, a collection of cells *(e.g.,* cultured cells), a tissue sample, a biopsy, or an organism. Biological samples also include blood and blood products (e.g., plasma) and other biological fluids (e.g., tears, sweat, saliva and related fluids, urine, tears, mucous, and so forth). Tissue samples can be from any organ or tissue in the body, include heart, liver, muscle, adipose, brain, lung, testes, and brain.

Biological samples may be from individual subjects (e.g., animals, such as humans, mice, rats, monkeys, chickens, cats, dogs, pigs, horses, cows, fruit flies, or worms) and/or archival repositories. The samples may be acquired directly from the individuals, from clinicians (for instance, who have acquired the sample from the individual), or directly from archival repositories.

Informatics: A global term used to describe a collection of modern, usually "high throughput" and computer-based scientific techniques that provide, generate, accumulate, and/or particularly analyze information about the genotypic and/or phenotypic and/or metabolic state of a cell or organism. Such techniques include genomic analyses and proteomic analyses, as well as metabolomic analyses. Informatics represents a subtle, but significant, shift in perspective among biologists. Whereas historically, scientists were accustomed to simplifying their systems to make metabolic interpretations, informatics allows scientists to embrace biological complexity and to make metabolic or phenotypic inference on the basis of as much information as possible. Genomics has brought to us the concept of high throughput science, and as a result, it has demonstrated the power of non-targeted and unbiased data acquisition. Although non-targeted data acquisition is uncommon in metabolite analysis, it does not violate the hypothesis-oriented procedure for scientific study. Rather, high-throughput and non-targeted data acquisition simply allows scientists to test their specific hypotheses on a larger, non-biased dataset. This investigative process functions differently than in a traditional reductionist approach, where experiments are designed to address single questions. Informatics focuses on obtaining accurate data that can be integrated with other datasets so that future hypotheses can be tested on a database *in silico* rather than at the laboratory bench. This method of investigation is suited to genomics, where sequences from disparate sources are integrated easily into one database because the genetic code is essentially universal. Because metabolomic data is influenced by the environment, and can be different depending on the time and conditions under which the sample is taken, a metabolomic database involves providing for considerably more complexity than is seen in a genomic database.

Lipid: As used herein, the term lipid refers to a class of water-insoluble, oily or greasy organic substances, that are extractable from cells and tissues by nonpolar solvents, such as chloroform or ether. The most abundant kinds of lipids are the fats or triacylglycerols, which are major fuels for most organisms. Another class of lipids is the polar lipids, which are major components of cell membranes. The following table (Table 1) provides one way of grouping major types of lipids; these have been grouped according to their chemical structure:

**Table 1:**

| **Lipid type** | **Representative examples or sub-groups** |
|---|---|
| Triacylglycerols | |
| Waxes | |
| Phosphoglycerides | phosphatidylethanolamine |
| | phosphatidylcholine |
| | phosphatidylserine |
| | phosphatidylinositol |
| | cardiolipin |
| Sphingolipids | sphingomyelin |
| | cerebrosides |
| | gangliosides |
| Sterols and their fatty acid esters | *(see Table 3)* |

Lipid metabolites may also be broken down into other recognized classes, such as those shown in Table 2:

**Table 2:**

| **SCIENTIFIC NAME** | **ABBREVIATION** |
|---|---|
| Lyso-Phosphatidylcholine | LY |
| Sphingomyelin | SP |
| Phosphatidylcholine | PC |
| Phosphatidylserine | PS |
| Phosphatidylinositol | PI |
| Phosphatidylethanolamine | PE |
| Cardiolipin | CL |
| Free Fatty Acids | FFA |
| Monoacylglycerides | MAG |
| Diacylglycerides | DAG |
| Triacylglycerides | TAG |
| Cholesterol Esters | CE |
| Phosphatidic acids | PA |
| Phosphatidylglycerols | PG |
| CDP-diacylglycerols | CDP-DAG |
| Lysocardiolipin | LyCL |
| Lysophosphatidylethanolamine | LyPE |

Specific subclasses (or groups of classes) of lipids can be distinguished based on the position of the fatty acids on the lipid back bone. For instance, the following are positionally specific isomers of lyso-lipid classes: 1-acyl, 2-lyso-x (where x is PC, PS, PE, PI, PG, or PA); 1-lyso,2-acyl-x (here x is PC, PS, PE, PI, PG, or PA); 1-acyl, 2,3-lyso-monoacylglyceride; 1-lyso, 2-acyl, 3-lyso-monoacylglyceride; 1,2-acyl diacylglydceride; and 1,3-acyl diacylglyceride.

Also included in the term lipid are the compounds collectively known as sterols. Table 3 shows representative sterols.

**Table 3:**

| **SCIENTIFIC NAME** | **MOLECULAR FORMULA** | **COMMON NAME** |
|---|---|---|
| 5b-cholestan-3b-ol | C₂₇H₄₈O | coprostanol |
| 5a-cholestan-3b-ol | C₂₇H₄₈O | dihydrocholesterol |
| 5-cholesten-3b-ol | C₂₇H₄₆O | cholesterol |
| 5,24-cholestadien-3b-ol | C₂₇H₄₄O | desmosterol |
| 5-cholestan-25a-methyl-3b-ol | C₂₈H₄₂O | campesterol |
| 5-cholestan-24b-methyl-3b-ol | C₂₈H₄₂O | dihydrobrassicasterol |
| 5-cholesten-24b-ethyl-3b-ol | C₂₉H₅₀O | b-sitosterol |
| 5,22-cholestadien-24b-ethyl-3b-ol | C₂₉H₄₈O | stigmasterol |

Metabolite: A biomolecule that has a functional and/or compositional role (such as a component of a membrane) in a biological system, and which is not a molecule of DNA, RNA, or protein. Examples of metabolites include lipids, carbohydrates, vitamins, co-factors, pigments, and so forth. Metabolites can be obtained through the diet (consumed from the environment) or synthesized within an organism. Genes and proteins exist in large part to break down, modify, and synthesize metabolites. Metabolites are not only directly responsible for health and disease, but their presence in a biological system is the result of a variety of factors including genes, the environment, and direct nutrition. By profiling the metabolite composition of a biological sample, for instance using the methods described herein, data on genotype, metabolism, and diet can be obtained in great detail. This data can be linked to clinical information and used to identify the true biochemical basis for health and disease.

Lipids are perhaps the most important subset of metabolites, because dietary lipids and lipid metabolism are clearly linked to the incidence and progression of several major degenerative diseases, including heart disease, diabetes, obesity, auto-immunity, and chronic inflammation. Moreover, because lipids are the only major nutrients that survive digestion intact, highly accurate information on individual nutrition can be gained from a lipid metabolite profile. Thus, a lipid metabolomic approach provides information encompassing the entire spectrum of factors that influence disease.

Each fatty acid may be found as a component of any lipid class, and in such combination is a different metabolite than it is on its own (free) or as a component in any other lipid class. Thus, palmitoleic acid in cholesterol esters is a distinct metabolite from palmitoleic acid in triacylglycerides, and so on. By way of example, if a system is used in which lipids are categorized into 17 classes (as shown in Table 2), and there an analysis determines the concentration of 38 fatty acids and sterols are determined in each class, then 17 x 38, or 646 specific metabolite concentrations may be determined.

**Metabolomics**: Highly parallel acquisition, databasing, and analysis of metabolite levels in a biological sample. In some instances, the sample is obtained from a subject or individual currently experiencing or being maintained under one or more defined condition(s). There are several levels of metabolomics - these can be differentiated for instance based on the scope of the individual metabolite profile, where scope refers to the number or type of metabolites measured in the individual analysis. Thus, lipid metabolomics is the study or analysis of a set of individual lipid metabolites. The set of data produced from analysis of an individual sample is referred to herein as a individual **lipid metabolite/metabolic profile ("lipomic profile")** of that sample. Certain examples of lipid metabolite profiles include a highly comprehensive set of metabolite measurements (a profile) by multi-parallel analyses. The claims are limited to lipomic profiles.

The comparison of two metabolite profiles of similar scope (*i*.*e*., containing information about the same or a similar or overlapping set or subset of metabolites) from cells/tissues/subjects that have been differently treated, or that are genetically different or different based on disease state or condition, provides information on the metabolic effects of the difference.

A metabolome is a data set that includes levels of metabolites in a biological system (e.g., a cell, tissue, biological fluid, or whole subject) under specific conditions; a multidimensional metabolome includes such data from like samples over a variety of conditions (*e*.*g*., time points, treatment points, different drug or other treatments, and so forth).

Quantitative metabolomic data as discussed herein include molar quantitative data, mass quantitative data, and relational data by either moles or mass (mole % or weight %, respectively) for individual metabolites, or subsets of metabolites. Quantitative aspects of metabolomic samples may be provided and/or improved by including one or more quantitative internal standards during the analysis, for instance one standard for each lipid class (in a lipomic profile). Internal standards employed in the methods described herein enable true quantification of each fatty acid from each lipid class, whereas traditional lipid analysis methods produce data in either a percent-of-total format or as a mixed population of lipid metabolites. Provided internal standards are designed to reflect any loss of fatty acid due to oxidation, discrimination, or cross-contamination.

Using methods described herein, quantitative data can be integrated from multiple sources (for instance, samples generated from different labs, samples from different subjects, or merely samples processed on different days) into a single seamless database, regardless of the number of metabolites measured in each discrete, individual analysis.

**Metabolite fingerprint (or linked profile):** A distinct or identifiable pattern of metabolite levels, for instance a pattern of high and low metabolites of a defined set, such as a biogenerative pathway. In specific embodiments, the metabolite levels in the fingerprint are absolute metabolite concentrations. Metabolite fingerprints (also referred to as linked profiles, e.g., a disease-linked profile or toxin-linked profile) can be linked to a tissue or cell type, to a particular stage of normal tissue growth or disease progression, to a dietary limitation or supplementation, or to any other distinct or identifiable condition that influences metabolite levels (e.g., concentrations) in a predictable or associatable way. Metabolite fingerprints can include relative as well as absolute levels of specific metabolites, but absolute levels (e.g., concentrations) are preferred in many embodiments. Specific examples of metabolite fingerprints are lipid metabolite fingerprints.

**Pharmaceutical/therapeutic agent:** Any agent, such as a protein, peptide (*e*.*g*., hormone peptide), other organic molecule or inorganic molecule or compound, or combination thereof, that has one or more effects on a biological system, such as a desired therapeutic or prophylactic effect when properly administered to a subject.

**Quantified metabolic profile:** A set of quantified measurements of one or more metabolites. The profile usually contains more than one quantified measurements for a metabolite and provides a metabolic snap shot of a condition. Specific examples of quantified metabolic profiles are specific for a condition to which an organism is subject, such as a genotype, for instance a knockout of a specific gene; a dietary limitation or supplementation; a disease or disease state; a treatment with a compound, for instance a drug, toxin, suspected toxin, pharmaceutical agent, or t compound that is a candidate for a pharmaceutical agent, and so forth.

Quantified measurement of a metabolite: A measurement of the concentration of a metabolite, obtained by using an internal standard for the metabolite. The measurement is usually readily comparable with any other measurements of the metabolite, e.g., from a different sample from a same or different organism, which different organism is subject to the same or a different condition, or samples generated using a different method or approach for obtaining the measurements. The quantified measurements can be integrated from multiple sources (whether it is work from different labs, samples from different subjects, or merely samples processed on different days) into a single database, regardless of the number of metabolites measured in each discrete, individual analysis. For example, quantified measurements of a lipid generally include measurements of the concentration of the lipid within each lipid class using one or more internal standards for each lipid class. The measurements can be compared with any other measurements of the lipid regardless how the measurements were obtained and can be integrated into one database readily searchable for useful indications or patterns.

**Subject**: Living multi-cellular vertebrate organisms, a category that includes both human and non-human mammals.

Unless otherwise explained, all technical and scientific terms used herein have the same meaning as commonly understood by one of ordinary skill in the art to which this invention belongs. The singular terms "a," "an," and "the" include plural referents unless context clearly indicates otherwise. Similarly, the word "or" is intended to include "and" unless the context clearly indicates otherwise. Hence "comprising A or B" means include A, or B, or A and B. It is further to be understood that all base sizes or amino acid sizes, and all molecular weight or molecular mass values, given for metabolites, nucleic acids or polypeptides are approximate, and are provided for description. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including explanations of terms, will control. In addition, the materials, methods, and examples are illustrative only and not intended to be limiting.

### III. Overview of Several Embodiments

One embodiment includes the generation of a quantitative lipid metabolomic database, which includes generating a plurality of quantitative lipid metabolite profiles from a plurality of biological samples and assembling the plurality of lipid metabolite profiles into a database. Biological samples for such methods may be selected from individual subjects and/or archival repositories, and may be acquired directly from individuals, from clinicians, or from archival repositories directly. In specific examples, the biological samples are taken from animals, for instance humans, mice, rats, monkeys, chickens, cats, dogs, pigs, horses, cows, fruit flies, or worms. In some embodiments, the generation of a quantitative lipid metabolite profile involves performing quantitative lipid metabolite analysis on a biological sample to generate a lipomic profile for the sample, entering the lipomic profile into one or more tables (for instance, a table on a computer), and repeating these steps a plurality of times. The plurality of data entries in the table(s) is a lipomic database.

Also provided are methods of permitting (for instance, for a fee) access to the metabolomic profile databases described herein. Examples of such methods involve embodiments in which access is through a computer interface, for instance from a remote computer across the Internet to the computer that contains the database itself.

The generation of quantitative lipomic data includes separating a biological sample into fractions based on a plurality of lipid classes wherein at least one quantitative internal standard is included for each lipid class, and measuring the quantity of a plurality of lipid metabolites in each of the fractions wherein the quantitative measurements of the plurality of lipid metabolites in the fractions are stored as a quantitative lipid metabolite profile. Either separating or measuring in these methods may involve a chromatographic method, such as thin-layer, gas and/or liquid chromatography. The plurality of lipid classes may include, for instance, phospholipids, glycerides, and other lipids. An alternative division of lipids into class may be as follows: lyso-phosphatidylcholines, sphingomyelins, phosphatidylcholines, phosphatidylserines, phosphatidylinositols, phosphatidylethanolamines, cardiolipins, free fatty acids, monoacylglycerides, diacylglycerides, triacylglycerides, and cholesterol esters.

In the methods described herein, lipid metabolites may include tetradecanoic acid, pentadecanoic acid, hexadecanoic acid, heptadecanoic acid, octadecanoic acid, eicosanoic acid, docosanoic acid, tetracosanoic acid, 9-tetradecenoic acid, 9-hexadecenoic acid, 11-octadecenoic acid, 9-octadecenoic acid, 11-eicosenoic acid, 5,8,11-eicosatrienoic acid, 13-docosenoic acid, 15-tetracosenoic acid, 9,12,15-octadecatrienoic acid, 6,9,12,15-octadecatetraenoic acid, 11,14,17-eicosatrienoic acid, 8,11,14,17-eicosictetraenoic acid, 5,8,11,14,17-eicosapentaenoic acid, 7,10,13,16,19-docosapentaenoic acid, 4,7,10,13,16,19-docosahexaenoic acid, 6,9,12,15,18,21-tetracoshexaenoic acid, 9,12-octadecadienoic acid, 6,9,12-octadecatrienoic acid, 11,14-eicosadienoie acid, 8,11,14-eicosatrienoic acid, 5,8,11,14-eicosicatetraenoic acid, 13,16-docsadienoic acid, 7,10,13,16-docosicatetraenoic acid, 4,7,10,13,16-docosapentaenoic acid, 9-trans-hexadecenoic acid, 9-trans-octadecenoic acid, 8-eicosaenoic acid, 5-eicosaenoic acid, plasmalogen fatty acids, 5b-cholestan-3b-ol, 5a-cholestan-3b-ol, 5-cholesten-3b-ol, 5,24-cholestadien-3b-ol, 5-cholestan-25a-methyl-3b-ol, 5-cholestan-24b-methyl-3b-ol, 5-cholesten-24b-ethyl-3b-ol, or 5,22-cholestadien-24b-ethyl-3b-ol, for instance. Individual fatty acids may be found as a component of any lipid class, and in such combination is a different metabolite than it is on its own (free) or as a component in any other lipid class. Thus, palmitoleic acid in cholesterol esters is a distinct metabolite from palmitoleic acid in triacylglycerides, and so on.

Further provided embodiments are methods for presenting analysis of a plurality of individual lipid metabolite profiles, which methods involve designating the plurality of individual metabolite profiles (for instance, from within a cumulative database of such profiles), identifying a plurality of differences or similarities in measured lipid metabolites between the plurality of quantitative lipid metabolite profiles, and displaying the plurality of differences or similarities in measured lipid metabolites between the plurality of individual metabolite profiles. This displaying comprises displaying a heat map, so as to enable the user an easy comparison between the plurality of lipid metabolite profiles. In specific examples of such embodiments, the displaying generates a web page for viewing.

Further provided methods include methods of determining a metabolic effect of a condition (such as a genotype, for instance a knockout of a specific gene; a dietary limitation; a disease or disease state; a treatment with a compound, for instance a drug, toxin, suspected toxin, pharmaceutical agent, or compound that is a candidate for a pharmaceutical agent) on a subject. Examples of such methods involve subjecting the subject to the condition, taking at least one biological sample from the subject (usually after they are subjected to the condition), analyzing the biological sample to produce a quantitative lipomic profile for the subject, comparing the quantitative lipomic profile for the subject with a quantitative control lipomic profile, and drawing conclusions about the metabolic effect of the condition based on differences or similarities between the quantitative lipomic profile from the subject and the quantitative control lipomic profile. The control lipomic profile may be for instance a compiled lipomic profile assembled from a plurality of individual lipomic profiles, or a pre-condition (e.g., pre-treatment) lipomic profile from the subject.

Specific examples of such methods are methods of determining the effectiveness of drug or treatment in a subject, for instance treatment with a hormone pr a drug or other treatment that relates to controlling obesity or diabetes. Generally, in these methods a biological sample obtained from a treated subject is analyzed to produce a test lipomic profile for the subject. This test lipomic profile for the subject is compared with a control lipomic profile (such as the control lipomic profiles discussed above), and conclusions are drawn about the effectiveness of the drug or treatment based on differences or similarities between the test lipomic profile and the control lipomic profile.

Another embodiment involves a database, where the database containing a profile table including a quantified lipid metabolic profile from a biological sample from an individual having a condition, wherein the quantified lipid metabolic profile includes a quantified measurement of a lipid metabolite (or more than one metabolite) and wherein the quantified measurement is obtained using an internal standard (such as those described herein) for the lipid metabolite so that the quantified measurement is integratable into a database.

Also described herein are databases that further include a sample item table including a sample record for the quantified lipid metabolic profile, and a condition item table including a condition record for the quantified lipid metabolic profile. Other specific examples of dabatases comprise a filter item table including a filter of quantified metabolic profile for a desired condition.

It is contemplated that, in some embodiments, biological samples in this context will include a biological fluid or tissue sample. Biological samples in some embodiments are selected from individual subjects or archival repositories, or some of both, or from animal models. In some examples, at least some of the biological samples used to generate the database are samples taken from an animal, for instance, a human, mouse, rat, monkey, chicken, cat, dog, pig, horse, cow, fruit fly, or worm. Specific databases contain profiles generated from biological samples from different species, different analysis methods, etc.

In addition, it is specifically contemplated that some samples are obtained from an organism that is subject to a condition. For instance, the condition can include a trait (such as a genotype, for instance a genetic knockout or other mutation) of the organism from which the biological sample is obtained; a dietary limitation or supplementation; a disease or disease state; application of a toxin or suspected toxin; application of a pharmaceutical or therapeutic agent or candidate agent to the organism; an increase in exercise, a decrease in exercise, or a change in an exercise regimen of the subject; or some combination of these circumstances.

In particular embodiments, the databases contains lipid metabolite data, wherein at least one quantified lipid metabolite is selected from the group consisting of tetradecanoic acid, pentadecanoic acid, hexadecanoic acid, heptadecanoic acid, octadecanoic acid, eicosanoic acid, docosanoic acid, tetracosanoic acid, 9-tetradecenoic acid, 9-hexadecenoic acid, 11-octadecenoic acid, 9-octadecenoic acid, 11-eicosenoic acid, 5,8,11-eicosatrienoic acid, 13-docosenoic acid, 15-tetracosenoic acid, 9,12,15-octadecatrienoic acid, 6,9,12,15-octadecatetraenoic acid, 11,14,17-eicosatrienoic acid, 8,11,14,17-eicosictetraenoic acid, 5,8,11,14,17-eicosapentaenoic acid, 7,10,13,16,19-docosapentaenoic acid, 4,7,10,13,16,19-docosahexaenoic acid, 6,9,12,15,18,21-tetracoshexaenoic acid, 9,12-octadecadienoic acid, 6,9,12-octadecatrienoic acid, 11,14-eicosadienoic acid, 8,11,14-eicosatrienoic acid, 5,8,11,14-eicosicatetraenoic acid, 13,16-docsadienoic acid, 7,10,13,16-docosicatetraenoic acid, 4,7,10,13,16-docosapentaenoic acid, 9-trans-hexadecenoic acid, 9-trans-octadecenoic acid, 8-eicosaenoic acid, 5-eicosaenoic acid, plasmalogen fatty acids, 5b-cholestan-3b-ol, Sa-cholestan-3b-ol, 5-cholesten-3b-ol, 5,24-cholestadien-3b-ol, 5-cholestan-25a-methyl-3b-ol, 5-cholestan-24b-methyl-3b-ol, 5-cholesten-24b-ethyl-3b-ol, and 5,22-cholestadien-24b-ethyl-3b-ol, each as a compound or a component of a lipid molecule.

Also encompassed herein is a database wherein the quantified metabolic profile includes a quantified measurement of a lipid in a lipid class. For instance, the quantified measurement of a lipid in a lipid class is in some instances obtained using an internal standard for the lipid class.

In some instances, a quantified lipid is selected from the group consisting of fatty acid 16:0, 18:0, 16:1n7; 18:1n7; 18:1n9; 18:3n3; 20:5n3; 22:5n3; 22:6n3; 18:2n6; 18:3n6; 20:3n6; and 20:4n6, each as a compound or a component of a lipid molecule. Other examples of lipids include a sterol selected from the group consisting of Sb-cholestan-3b-ol, 5a-cholestan-3b-ol, 5-cholesten-3b-ol, 5,24-cholestadien-3b-ol, 5-cholestan-25a-methyl-3b-ol, 5-cholestan-24b-methyl-3b-ol, 5-cholesten-24b-ethyl-3b-ol, and 5,22-cholestadien-24b-ethyl-3b-ol, each as a compound or a component of a lipid molecule.

Lipid classes include lyso-phosphatidylcholine, sphingomyelin, phosphatidylcholine, phosphatidylserine, phosphatidylinositol, phosphatidylethanolamine, cardiolipin, free fatty acids, monoacylglycerides, diacylglycerides, triacylglycerides, and cholesterol esters, for instance.

Representative examples of such internal standards are provided herein, as is teaching to make internal lipid standards more generally. A particular embodiment is a database as described above, wherein at least one internal standard is selected from the group consisting of diheptadecanoyl phosphatidylcholine, dipentadecaenoyl phosphatidylethanolamine, tetraheptadecenoyl cardiolipin, diheptadecenoyl phosphatidylserine, pentadecenoyl sphingomyelin, heptadecanoyl lysophosphatidylcholine, tripheptadecaenoyl glyceride, pentadecaenoic acid, heptadecanoic cholesterol ester and free fucosterol. In other specific embodiments, the internal standard is heptadecanoic 1-heptadecanoyl-2-lyso-phosphatidycholine for the lipid class of lysophospholipids, N-pentadecenoyl-D-erythro-sphingosylphorylcholine for the lipid class of sphingomyelin, 1,2 diheptadecanoylphosphatidylcholine for the lipid class of phosphatidylcholine, 1,2-diheptadecenoylphosphatidylethanolamine for the lipid class of phosphatidylethanolamine, 1,2-diheptadecenoylphosphatidylserine for the lipid class of phosphatidylserine, pentadecaenoic acid for the lipid class of free fatty acids, triheptadecaenoic acid for the lipid class of triacylglycerides, 1,1',2,2'-tetraheptadecaenoyl cardiolipin for the lipid class of cardiolipin, cholesteryl heptadecanoate for the lipid class of cholesterol esters and stigmasterol for the lipid class of free sterols.

Another embodiment involves a user interface for operatively working with a processor to affect operation of a database as provided herein, where the user interface includes means for providing settings for selecting a set of samples, means for providing settings for selecting a set of conditions, means for providing settings for selecting a set of lipid metabolites, and means for displaying quantified lipid metabolic profiles corresponding to the selected samples and conditions, wherein each displayed quantified lipid metabolic profile consists of the quantified measurements of the selected lipid metabolites. Optionally, the user interface can further include a display area which displays the value of a quantified measurement of a lipid metabolite within the quantified lipid metabolic profiles of the selected samples and conditions. Optionally, the user interface can further include means for comparing quantified lipid metabolic profiles corresponding to a first set of selected samples and conditions to the quantified lipid metabolic profiles corresponding to a second set of selected samples and conditions, and means for displaying the comparison.

Specific examples of the encompassed user interfaces include, for a plurality of lipid metabolites, a presentation of an observed quantity of at least one lipid metabolite for a first biological sample with respect to an observed quantity of the at least one lipid metabolite for a second biological sample, wherein the presentation is operable to accept a user indication that further information is desired with respect to a selected lipid metabolite.

Specific internal standards and internal standard compositions, which often contain a mixture of two or more internal standards, are also provided. By way of example, another embodiment is an internal standard composition for lipid analysis of a sample, comprising a plurality of lipid species, wherein at least one lipid species comprises at least one monounsaturated fatty acid of formula N:1nR, wherein N is an odd integer equal to or larger than three, wherein R is any integer equal to or less than N-1, and wherein at least one of the plurality of lipid species is a free fatty acid, a sphingomyelin, a cardiolipin, a phosphatidylethanolamine, a phosphatidic acid, a phosphytidylcholine, a phosphatidylserine, a phosphatidylinositol, a phosphatidylglycerol, a monoacylglyceride, a diacylglyceride, a triacylglyceride, a sterol ester, or a lysophospholipid. In specific examples of these compositions, each lipid species comprises at least one such monounsaturated fatty acid.

In particular example internal standard compositions, at least one of the monounsaturated fatty acids in the standard is not present in the sample. In examples of such compositions, each of the monounsaturated fatty acids is not present in the sample.

Particular examples of these internal standard compositions will include at least one lipid species having at least one monounsaturated fatty acid, wherein N is 3, 5, 7, 9, 11, 13, 15, 17, 19, 21, 23, or 25.

Optionally, each of the plurality of lipid species in the internal standard composition represents a specific (for instance, a different) lipid class. In some instances, each of the plurality of lipid species in an internal standard is present in the composition at a concentration equivalent to (e.g., with an order of magnitude) the concentration of a sample lipid species (for instance, the most abundant, second most abundant, third most abundant, and so forth) from the same lipid class as represented by that lipid species. By way of example, the internal standard compositions may include at least three lipid species, at least three lipid species, at least four lipid species, at least five lipid species, at least eight lipid species, or at least three ten species or more.

In particular example compositions, at least one of the lipid species is a lysophospholipid, and the lysophospholipid has the formula 1-acyl,2-lyso-M or 1-lyso,2-acyl-M, and where M is phosphytidylcholine, phosphatidylserine, phosphatidylethanolamine, phosphatidylinositol, phosphatidylglycerol, or phosphatidic acid.

In still other particular example compositions, the lipid classes comprise lyso-phosphatidylcholines, sphingomyelins, phosphatidylcholines, phosphatidylserines, phosphatidylinositols, phosphatidylethanolamines, cardiolipins, free fatty acids, monoacylglycerides, diacylglycerides, triacylglycerides, cholesterol esters, phosphatidic acids, phosphatidylglycerols, CDP-diacylglycerols, lysocardiolipins, lysophosphatidylethanolamines, or two or more thereof.

Also provided is an internal standard for phosphatidylethanolamines, phosphatidic acids, phosphytidylcholines, phosphatidylserines, phosphatidylinositols, phosphatidylglycerols, diacylglycerides, or triacylglycerides, comprising a first fatty acid of formula N:0 in the sn-1 position and a second fatty acid of formula M:Y in the sn-2 position, where Y is an integer greater than 0. One specific example is an internal standard for phosphatidylethanolamines, wherein the internal standard comprises a phosphatidylethanolamine that comprises the first fatty acid and the second fatty acid. Another specific example is an internal standard for phosphatidic acids, wherein the internal standard comprises a phosphatidic acid that comprises the first fatty acid and the second fatty acid. Still another example is an internal standard for phosphytidylcholines, wherein the internal standard comprises a phosphytidylcholine that comprises the first fatty acid and the second fatty acid. Yet a further example is an internal standard for phosphatidylserines, wherein the internal standard comprises a phosphatidylserine that comprises the first fatty acid and the second fatty acid. Another example is an internal standard for phosphatidylinositols, wherein the internal standard comprises a phosphatidylinositol that comprises the first fatty acid and the second fatty acid. Yet another example is an internal standard for phosphatidylglycerols, wherein the internal standard comprises a phosphatidylglycerol that comprises the first fatty acid and the second fatty acid. Another provided example is an internal standard for diacylglycerides, wherein the internal standard comprises a diacylglyceride that comprises the first fatty acid and the second fatty acid. Still another example is an internal standard for triacylglycerides, wherein the internal standard comprises a triacylglyceride that comprises the first fatty acid and the second fatty acid. Optionally, such an example internal standard for triacylglycerides further includes a third fatty acid that is different from the first fatty acid and the second fatty acid.

Another embodiment is an internal standard for triacylglycerides or cardiolipins, comprising a first fatty acid of formula N:X at a first position, a second fatty acid of formula M:Y at a second position, and a third fatty acid of formula O:Z at a third position, wherein N:X, M:Y, and O:Z are different from each other. In some examples, the first position is sn-1 and X is 0. In anther example, at least Y or Z is 1, and in specific examples, both Y and Z are 1, For instance, in one particularly contemplated example of such an internal standard, N:X is 17:0, M:Y is 19:1, and O:Z is 19:1 and wherein the first position is sn-1, the second position is sn-2, and the third position is sn-3. In another, N:X is 17:0, M:Y is 19:1, and O:Z is 19:2 and wherein the first position is sn-1, the second position is sn-2, and the third position is sn-3. In still other examples, the internal standard is an internal standard for triacylglycerides, wherein the internal standard comprises a triacylglyceride that comprises the first fatty acid, the second fatty acid, and the third fatty acid. In yet another example, it is an internal standard for cardiolipins, wherein the internal standard comprises a cardiolipin that comprises the first fatty acid, the second fatty acid, and the third fatty acid. For instance, in such an internal standard for cardiolipins, the first position is sn-1, the second position is sn-2, and the third position is either sn-1' or sn-2'. By way of example, in one such internal standard the third position is sn-1', and X and Z are 0.

Also provided is an internal standard composition for lipid analysis of a sample, comprising a plurality of lipid species, wherein at least one lipid species comprises at least one polyunsaturated fatty acid of formula N:lnR, wherein N is an even integer equal to or larger than six (for instance, 6, 8,10,12,14,16,18,20,22,24, or 26), wherein R is any integer equal to or less than N-1, and wherein the desaturations occur in positions different from the positions of desaturations in fatty acids present in the sample, and wherein at least one of the plurality of lipid species is a free fatty acid, a sphingomyelin, a cardiolipin, a phosphatidylethanolamine; a phosphatidic acid, a phosphytidylcholine, a phosphatidylserine, a phosphatidylinositol, a phosphatidylglycerol, a monoacylglyceride, a diacylglyceride, a triacylglyceride, a sterol ester, or a lysophospholipid. In specific examples of such internal standard compositions, each lipid species comprises at least one such polyunsaturated fatty acid.

In specific examples of these internals standard compositions, each of the plurality of lipid species represents a different lipid class. For instance, such compositions can contain at least three lipid species, at least four lipid species, at least five lipid species, at least eight lipid species, at least ten lipid species, or more.

In still other specific examples of the internal standard compositions, each of the plurality of lipid species is present in the composition at a concentration equivalent to the concentration of a sample lipid species from the same lipid class as represented by that lipid species.

Also provided are specific internal standard compositions, wherein at least one of the polyunsaturated fatty acids is not present in the sample. In further examples, each of the polyunsaturated fatty acids in the internal standard is not present in the sample.

By way of specific example, at least one of the lipid species in the internal standard compositions is a lysophospholipid, and the lysophospholipid has the formula 1-acyl,2-lyso-M or 1-lyso,2-acyl-M, and where M is phosphytidylcholine, phosphatidylserine, phosphatidylethanolamine, phosphatidylinositol, phosphatidylglycerol, or phosphatidic acid. In other specific examples, the lipid classes included in the internal standard composition include lyso-phosphatidylcholines, sphingomyelins, phosphatidylcholines, phosphatidylserines, phosphatidylinositols, phosphatidylethanolamines, cardiolipins, free fatty acids, monoacylglycerides, diacylglycerides, triacylglycerides, cholesterol esters, phosphatidic acids, phosphatidylglycerols, CDP-diacylglycerols, lysocardiolipins, lysophosphatidylethanolamines, or two or more thereof.

The internal standards described in this disclosure, including particular single internal standard molecules or combinations thereof or compositions containing such, can be used with the methods provided herein, particularly with the generation of quantitative lipomic data.

### IV. Metabolomics

The vast potential of genomics and bioinformatics to identify genes that cause disease by investigating whole-genome databases is accepted. By comparing the analysis of an individual's genotype with a genomic database, medicine is expecting to personalize health care by providing drugs tailored to individual genotype. This same bioinformatic approach, when applied to the study of human metabolites, has the potential to identify and validate targets to improve personalized health through nutrition, pharmacology, environment, physical activity, and/or gene therapy. Advances in high-throughput analytical chemistry and computing technologies make the creation of a vast database of metabolites possible for several subsets of metabolites including lipids and organic acids.

In creating integrative databases of metabolites for bioinformatic investigation, the current concept of single biomarker measurements must be expanded in three dimensions in order to:
(1) include a highly comprehensive set of metabolite measurements (a profile) by multi-parallel analyses;
(2) measure individuals as a function of time rather than simply in the fasted state; and
(3) integrate these metabolic profiles with genomic, expression and proteomic databases.

Substantial databases of metabolite concentrations will be predictive resources to quantify the relationship between metabolites and health. An overview of one way in which a metabolomic database can be used is shown in Figure 1. In this schematic drawing, quantitative analysis is performed to assess and measure the amounts of metabolites in a biological sample. The output of the analysis is subjected to optional transformation through one or more calculation processes, providing a set of numeric results. For instance, if the analysis is a quantitative gas chromatograph, the area under the curve can be measured and the relative area of each peak determined. These relative areas can be converted into absolute amounts for each individual metabolite measured by the inclusion of control compounds in the analysis, as described herein. The raw and/or processed data are entered into a database, for instance a cumulative database that contains the results from a multitude of different analyses. This database can be queried in order to search for specific datasets from within the database, and filters (such as those provided herein) can be used to produce limited output in interpretable forms. Such forms may be user interfaces that permit continued interaction with the database, and/or that permit access to more information than the raw or processed results of individual analyses or collections thereof. In certain embodiments, the output from such a metabolomic database may be graphical or statistical.

Quality control triggers may be included within the database, which flag samples that are outside of expected or predicted limits, or which otherwise trip a trigger so that the user of the database (and/or the individual entering the data, or a third party) is made aware of that specific sample. In specific embodiments, the tripping of such a trigger will indicate that the corresponding sample is in someway suspect, and the analysis for that sample may be repeated.

The application of an informatic approach to the study of metabolites in individuals represents an important advance. Scientists currently view their goal as ultimately reductionist and strive to identify the single best biomarker that reflects phenotype. However, single biomarkers have shown very limited success in predicting chronic disease. This has led the inventors to the realization that there is a need for more global and integrated approaches for assessing metabolism. Thus, the study of metabolites must be redefined in parallel with genomic and proteomic analyses, as the means to allow researchers to measure a large number or even an entire set of metabolites. The entire metabolome, with all of its individual concentrations and quantitative intra-relationships forms the metabolic basis of a phenotype. Therefore, only a metabolomic approach can accurately assess the complex role of metabolites in defining individual health.

In part, the reluctance to study metabolism within the framework of informatics arises from the inherent complexity of metabolite profiling. Although expression analysis and proteomics are responsive to the environment and are thus more complex than genomics, they are constrained, at least in theory, by a factor of the number of genes present in an organism. The overall metabolome is not confined to the products of genes, and thus, the metabolome represents a potentially massive inclusive set of compounds. Further, a metabolite profile for a single individual is neither constant among individual cells, nor is it stable over time. Implementing a metabolomic research strategy involves planning for considerable complexity. This disclosure provides methods for generating metabolomic profiles for individual samples, for sets or subsets of the available metabolites, and methods of assembling such profiles into integrated, comprehensive, minable databases.

The ultimate application of these approaches, of course, is to generate knowledge of metabolism that is faithful to the overall phenotypes that accurately reflect health, predispositions to disease, or other health outcomes. In nutritional terms, for example, understanding the variation in metabolic responses to diet is the goal of the science of nutrition. Before embarking on wholesale renovations of agricultural products for nutritional improvement, metabolomics is uniquely qualified to address the questions that must be answered to succeed. Although the development of this technology is likely to be driven by human health concerns, rapid analysis of lipids and other metabolite classes can be used in the support of a variety of topics including plant and animal breeding, characterization of transgenic crops, and fundamental science. Metabolomics will be a part of the future of biotechnology, nutrition, and agriculture.

### V. Lipid Metabolomics

This disclosure focuses on analysis of lipid metabolites, generation of lipid metabolite profiles, lipid metabolomic databases, and the information that can be mined from such profiles and databases. In particular, methods are provided for developing a metabolomic database capable of producing predictive and diagnostic profiles of disease.

Figure 2 provides an overview of a lipid metabolomic analysis system. Chromatographic data is entered into a database, which can be mined for desired information and presented in the form of a graphical interface a heat map, as shown in the second panel of Figure 2). Such an interface may be optionally provided in interactive form, for instance on a computer system, or remotely across the Internet or another computer communication system. Data mined from the cumulative lipid metabolite database can be used, for instance, for clinical or diagnostic testing (e.g., for a propensity to obesity or another biological condition that impacts or is impacted by lipid metabolism), or to identify specific metabolic targets of drugs, as described in more detail herein.

Present analytical methods, including those disclosed herein, can produce a spectrum of data easily developed into a metabolomic database. For instance, fatty acids, glycerolipids, sterols, and numerous bioactive lipid mediators (including products of epoxygenase, lipoxygenase and cyclooxygenase pathways) are quantifiable in biological samples. Thus, as demonstrated clearly herein, a few parallel analyses are capable of defining an essentially complete lipid profile of a sample.

Lipids are an attractive subset of metabolites for metabolomic applications. In addition to their ubiquitous cellular functions as structural, energetic, and bioactive signaling molecules, lipids are reflective of both diet and metabolism. The major fatty acids in human metabolism and the enzymes that modify them are depicted in Figure 3. Fatty acids are an interesting subject matter for metabolomics because they are the only major macronutrients to survive digestion intact, and yet humans possess the biochemical machinery to process dietary fatty acids further into new forms of fatty acid. As a result, the fatty acid composition of tissues and fluids reflects the influence of both diet and metabolism. By quantifying the fatty acids present in human plasma, for example, a researcher could determine the dietary preferences of that individual. Alternatively, and perhaps more interestingly, a researcher could assay endogenous lipid metabolism by comprehensive lipid analysis, because every lipid substrate and product is measured simultaneously from a single sample. Thus, a unique aspect of lipid metabolomic analysis is that the information yielded by an experiment reflects the ultimate expression of genomics, proteomics, and environment as a lipid metabolome.

Because current technology allows for the comprehensive analysis of lipid composition in a sample, metabolic interpretations can be extended to the activities of the enzymes that modify lipids. Quantitative analysis of fatty acid concentration provides data on not only the fatty acids but also on the relative activities of the desaturases and elongases that modify them. Moreover, a quantitative analysis of fatty acids from individual glycerolipid classes yields data on the mass of each glycerolipid class, thereby enabling the investigation of pathways involved in glycerolipid metabolism. The ability to not only profile diseases, but also to identify the complex metabolic dysregulations involved in that disease, using the methods provided herein is a major advance for medicine.

The utility of metabolite profiling is not limited to making assessments about the status of individuals. Particularly, one advantage of metabolomics is the potential to use a metabolomic database as a tool for *in silico* investigations. The availability of such databases will be particularly helpful for applying bioinformatic approaches to nutrition, pharmacology, and toxicology, because once a metabolic profile is developed for a specific nutritional or otherwise affected state, it can be compared with the metabolomic database to determine the relationships among diet, drugs, toxins, treatments, genotype, and phenotype. The ability to mine large databases *in silico* will be an advantage of metabolomics to nutrition, because testing every conceivable nutrient by single clinical trials is not possible.

Moving from single biomarkers to metabolomic analysis is a necessary step inasmuch as many approaches to lowering the unilateral risk of one disease in an individual simply increase the risk of another disease in that individual. A pertinent example of this problem is the change in nutritional recommendations from high fat to high carbohydrate diets. It is widely understood that high fat diets increase serum low-density lipoproteins and thus the risk for cardiovascular disease in most individuals; however, high carbohydrate diets increase serum triacylglycerides (Kasim-Karakas et al., Am. J. Clin Nutri. 71:1439-1447, 2000) and the risk for cardiovascular disease in a subset of the population, particularly some women (Liu et al., Am. J. Clin. Nutri. 71:1455-14612000). By measuring every metabolite involved in lipid metabolism, subtle differences in the predisposition or progression of disease among individuals will be elucidated. The broader and much more exciting aspect of this technology is thus the generation of metabolic profiles that are not simply markers for disease, but metabolic maps that can be used to identify specific genes or activities influential in the progression of disease or the maintenance of overall health. In this way, metabolomics is a subset of functional genomics. The value of genomic, expression, proteomic, and metabolomic databases in predicting phenotype will be enhanced dramatically by their horizontal integration into global bioinformatic databases.

### VI. Application of Lipid Metabolomics to Predictive Medicine

Relative to biomolecules, biochemical science has very few ways to quantify phenotype. Alternatively, medicine has, at its very core, a system for identifying, categorizing and recording phenotypic information about individuals. Because science has become exceedingly adept at quantifying large numbers of molecules at an astonishing rate of throughput, science and medicine should couple their expertise to develop this metabolite-phenotype relationship. By developing a database that (1) allows clinicians to input patient information and (2) allows high-throughput science to contribute analytical data, powerful new predictive and analytical tools are enabled.

The data from a comprehensive lipid analysis produce information useful for this purpose. The applications of a quantitative lipid database are myriad. In one variation, the data from comprehensive lipid analyses are used to generate biomarkers of a selected phenotype. These biomarkers are not, as traditionally defined, single measurements, but rather complex lipid metabolite profiles that include a large number of metabolites and even relations between metabolites. These profiles, when compared between experimental groups, generate a series of significant differences that can be used to construct reliable database filters. A database filter is essentially a way of discriminating a set of subset of data, and selecting this data from the database for instance for display or further analysis. Simple filters can comprise as few as one specified discriminating variable, for instance the gender of the individual providing the sample, or the age, or a treatment compound. More complex filters, using more than one discriminating variable at a time, are also contemplated. In specific instances, the filter can include a list of the most consistent and unique metabolite concentrations or interactions that exist between experimental groups (e.g., a filter can be based on a profile, such as a condition-linked profile). These differences and interactions are determined by standard statistical methods.

Database filters for specific phenotypes can serve many purposes. First, using discriminant analysis or an analogous statistical technique, a database filter can identify entries in a database that match a phenotype of interest. This is an essential element to metabolomics and informatics in general, because it allows scientist to query a database of individuals that were not specifically tested for the phenotype of interest.

A second purpose for creating a list of reliable and unique differences between experimental groups (a database filter) is to identify the points in the lipid metabolism pathways most closely linked with a phenotype. As an example of this approach, a researcher might perform an experiment to determine the complete lipid profile of patients with type II diabetes. These data would be recorded with all of the phenotypic and clinical information relevant to the patient in a database. At a later point in time, another researcher could generate metabolic profiles for individuals consuming different foods, such as dietary olive or fish oils, respectively, and enter this information into the same database. Both researchers would now have the ability to identify groupings of patients that match either diabetic or dietary profiles. Once the data are collected, it is a simple matter of asking the appropriate question *in silico* to determine if there are relations between dietary oil consumption and diabetes. Additionally, the identified differences act as clues for the metabolic basis of the effect.

There are innumerable advantages to an *in silico* approach such as outlined above, including increased statistical power, the avoidance of cumbersome financial and practical limitations to experimentation, and the ability to re-assess data as new information emerges. Subject matching, dataset selection, and the grouping of experimental sets can all be done through *in silico* querying. It is expected that unanticipated relationships between diet, metabolism, and phenotype will quickly emerge.

### VII. Metabolomics as Functional Genomics

Another aspect of the provided technology is the generation of metabolic profiles that are not simply markers for disease, but are metabolic maps that can be used to identify specific genes or biochemical activities that cause or influence a disease state. Metabolomics is in essence functional genomics from metabolite analysis. By defining the metabolic basis for phenotype using the techniques described, extraordinary opportunities to understand and treat diseases are provided. Much in the same way that gene chips allow researchers to observe the complex expression response to a stimulus, metabolomics enables observation of the complex metabolic interplay responsible for defining phenotype.

By extending this approach beyond the observation of individual metabolic dysregulations, medicine will begin to profile not single diseases, but health. As health is the proper balance of all vital metabolic pathways, comprehensive or metabolomic analysis lends itself to identifying metabolite distributions necessary for health. Comprehensive and quantitative analysis of lipids provides this degree of diagnostic power to researchers and doctors interested in mining metabolic profiles, and databases containing a plurality of such profiles, for biological meaning.

### VIII. Samples and Sample Processing

Any sample that contains or may contain the metabolites of interest can be used for the analyses provided herein. For instance, samples suitable for inclusion in a quantitative lipid metabolite database include plasma, serum, tissues or cells from plants, humans or research animals (including mouse, rat, non-human primate, pig, chicken or other). The samples may be those from plants, humans or other animals, which may optionally have been subjected to pharmacological, genetic, toxicological or nutritional intervention. In other embodiments, the samples are from humans or research animals expressing specific traits, for instance those suffering from a disease or condition, or displaying a level of athletic performance.

It is particularly contemplated herein that biological samples may be *in vitro* cell cultured samples, which have been subjected to differential treatment with drugs or potential drugs, or with any potentially useful pharmaceutical agent (for instance, which might be contemplated as being tested for use as a drug), or with a toxin or other stressor or organic or inorganic substance that might be expected to cause some change in the metabolome of the subject cell culture.

The processing of individual samples will be governed at least in part by what type of sample is used. Methods of harvesting biological samples are well known to those of ordinary skill in the art, and those appropriate for use with the provided methods are conventional. Methods for preparing the harvested samples for analysis will be influenced by the analysis being performed in order to quantify the metabolite(s) of interest. Those of ordinary skill in the art know systems that can be used to isolate (at least relatively) specified classes of molecules.

Optionally, biological samples for use in the provided methods can be stored prior to preparation and analysis, for instance by freezing, for instance under cryogenic conditions.

It is contemplated that sample preparation may be carried out by someone other than the party that carries out the analysis of metabolites in that sample. Thus, this disclosure includes systems in which a sample is harvested, processed at least to a point at which it can be shipped to a remote location, and then the processed (or partially processed) sample is transported to a facility at which the metabolites are assayed. By way of example, the samples may be transported while frozen.

Likewise, the treatment of subjects prior to harvesting of biological samples may be carried out at the same facility that harvests the sample, but this is not necessary for the methods described herein.

### IX. Individual Sample Analysis

Several aspects of lipid analysis have been modified, as provided herein, for use in lipid metabolomics. Currently, most fatty acid analyses are performed by gas chromatography, a technique that provides exquisite separation and quantification of analytes. However, researchers continue to report their results as a percentage of total fatty acids. Data in this format are not comparable between experiments, nor is it comparable between individual lipid classes within an experiment, and therefore are not integratable into a database. For example, a scientist interested in the metabolism of oleic acid could not determine the distribution of oleic acid among lipid classes in plasma from mole percentage or weight percentage data. For quantified measurements of lipid metabolomics as provided herein, data produced in each experiment is expressed as a concentration, for example, micrograms per milliliter, so that a consistent and comparable database of lipids can be assembled from multiple experiments.

In certain embodiments, the data includes quantitative measurements of the fatty acids that are organized (or can be organized) by lipid classes. Because lipid classes in some embodiments are separated prior to fatty acid quantification, the composition of a sample is determined in great detail. The results of a single analysis may include the mass or concentration of more than 35 individual fatty acids from each lipid class present in the sample. The total mass or concentration of the lipid class also may be quantified.

Separation and quantitative data can be produced via chromatography using many methods, such as gas and liquid chromatography, including high-performance liquid chromatography, thin layer chromatography, capillary and gel electrophoresis, and combinations of two or more of these methods. See, for instance, methods described in various text and reference analytic chemistry books, such as chapters 22-24 of Quantitative Chemical Analysis by D.C. Harris (W.H. Freeman and Co., 4th, 1995; ISBN 0-7167-2508-8). Choice of separation and quantitation methods may be influenced by the metabolites being measured.

By way of example, the following methods can be used for generating quantitative lipid metabolite data from biological samples. The chromatographic conditions, internal standard compositions and amounts, derivatization reactions, extraction conditions, sample amounts, and so forth can be varied by those of ordinary skill in the art. The following description provides an overview of certain non-limiting methods that can be used for analysis of lipid metabolites in a biological sample.

### A. Lipid Analysis

**Extraction**: The lipids from various samples, such as plasma, serum, tissue, or cells, can be extracted using a fluid extractant comprising a non-polar component and a polar component. By way of example, lipids are extracted from plasma, serum, tissues, and cells by the method of Folch et al. (J. BioL Chem. 226, 497-509, 1957). By way of example, about two hundred microliters of plasma or serum, or about 50 mg of tissues or cells are added to a homogenizer for a single analysis, though larger or smaller amounts can be used.

To each sample, the appropriate masses of internal standard such as those provided herein are added, as well chloroform:methanol (2:1 vol/vol). In general, the internal standards are compounds that share a lipid class with the target metabolites (*i*.*e*. an internal standard for triacylglyceride metabolites is itself a triacylglyceride), but have fatty acids as constituents that are not present in the sample being analyzed. An internal standard for any given lipid class is selected to behave sufficiently similarly to the target metabolites such that there is essentially no discrimination (selective loss or retention) of the internal standard relative to the target metabolites at any step of the analytical process before the analysis. The fatty acid moiety of the internal standard compound will also generally be different than the fatty acids present in the lipid class analyzed from the sample, so that the internal standard fatty acid can be separated completely from the target compound fatty acids by the analysis. According to specific provided embodiments, at least one unique internal standard is used for each class of lipid separated.

By way of example, the solution mixture consisting of sample, fluid extractant, and internal standard(s) is homogenized, for instance by twelve strokes with a ground-glass homogenizer. Following homogenization, potassium chloride (*e*.*g*., 1.8 ml of 0.01 M) is added, and the solution vigorously mixed. The organic fraction containing the lipids and the internal standard(s) is separated from the polar fraction of the mixture by centrifugation. The lipid extract can then be removed from the mixture and, as needed, concentrated under a stream of nitrogen in preparation for lipid class separation.

**Internal Standards**: Internal standards for use in the provided methods may take many forms. In certain embodiments, lipid classes that separate adjacent to each one another during lipid class separation have internal standards that contain fatty acids that are different than the fatty acids within the internal standard of the adjacent lipid class. This allows one to check for cross-contamination and complete separation of lipid classes by looking for the presence of the internal standard of one lipid class in the analysis of the adjacent lipid class.

In some embodiments, saturated fatty acids are employed as internal standards for the analysis of sphingomyelin, lyso-phospholipids (provided they are 1-acyl-2-lyso-phospholipids) and cholesterol esters, while saturates and monounsaturated fatty acids are used as internal standards for diacylglycerides, monoacylglycerides and free fatty acids.

Optionally, the internal standards provided by the present disclosure are added to each sample such that the fatty acids derived from the internal standard prior to the analysis are present at concentrations that approximate the second most concentrated fatty acid in the analyzed lipid class of the biological sample. This helps to ensure that the internal standard provides accurate data for quantifying the fatty acids and, provided the concentration of the sample is appropriate, that both the analytes and the internal standard induce a response from the detector that is within its linear and quantitative range.

Various fatty acids are particularly contemplated as internal standards, including fatty acid saturates, *e*.*g*., 3:0, 5:0, 7:0, 9:0, 11:0, 13:0, 15:0, 17:0, 19:0, 21:0, 23:0, 25:0, and 27:0, and fatty acid monounsaturates, *e*.*g*., 5:1, 7:1, 9:1,11:1, 13:1, 15:1, 17:1, 19:1, 21:1, 23:1, and 25:1. In particular embodiments, internal standards will include 17:0, 19:0, 15:1, 17:1 and 19:1.

In addition, polyunsaturated fatty acids may be used as internal standards, provided that they are odd-carbon numbered chains (e.g., 3:2, 5:2, 7:2, 9:2, 11:2, 13:2, 15:2, 17:2, 19:2, 21:2, 23:2, 25:2, and 27:2, as well as 5:3, 7:3, 9:3, 11:3, 13:3, 15:3, 17:3, 19:3, 21:3, 23:3, 25:3, 27:3, and so forth for additional unsaturations).

The position of unsaturated bond(s) within the fatty acid(s) of a standard can be varied to produce a large variety of internal standard compounds. For instance, 15:1n7 and 15:1n9 are distinct fatty acids that share highly similar physical properties. For instance, if 15:1 fatty acids provide the physical properties that best mimic lipid classes that also happened to separate next to each other during lipid class separation, one of these two fatty acids (15:1n7 and 15:1n9) could be used in one class, while the other fatty acid could be used in the second lipid class. Thus, because metabolite analysis is capable of separating 15:1n7 from 15:1n9, the degree of cross-contamination or separation of the two lipid classes could be determined. In general, it is useful if the position of the double bond(s) in the fatty acid(s) is unique relative to the composition of the biological sample, thus facilitating distinguishing these compounds in the final analysis and/or quantification.

In some embodiments, a mixture of internal standards is used to control different aspects of the analysis, e.g., positional specificity or compositional variation. For example, complex lipids that contain more than one fatty acid per molecule, such as phospholipids and triacylglycerides, typically contain defined types of fatty acids in specific positions on the lipid molecule. For instance, saturated fatty acids comprise more than 90% of the fatty acids on the sn-1 position (the first carbon on the glycerol backbone) of phosphatidylcholine in most biological samples, while unsaturated fatty acids comprise more then 95% of the fatty acids present in the sn-2 position of phosphatidylcholine. Thus, to improve the physical properties of an internal standard for phosphatidylcholine, it may prove useful to construct an internal standard molecule such that it contains a saturated fatty acid in the sn-1 position and an unsaturated fatty acid in the sn-2 position. This approach can be used to improve the physical properties of the internal standard to better match those of natural compounds.

Many types of chromatography can selectively deplete fatty acid molecules based on the number of double bonds present in the fatty acid, or on the number of carbons in the fatty acid. By constructing internal standards with a variety of fatty acids of varying unsaturation and chain length, these internal standards can control for these selectivities. For example, if internal standards are constructed with different fatty acids, e.g., with the different fatty acids present on the same glycerolipid molecule, such as triacylglyceride with a 17:0 on the sn-1 position, a 19:1 on the sn-2 position and a 19:2 on the sn-3 position, the extent of loss of fatty acids of varying unsaturation or chain length during analysis can be calculated and used to correct the final data for improved quantification. Thus panels of internal standards for each lipid class can be constructed with knowledge of the typical biological composition of the lipid class.

By way of specific example, this disclosure particularly contemplates internal standards including diheptadecanoyl phosphatidylcholine, dipentadecaenoyl phosphatidylethanolamine, tetraheptadecenoyl cardiolipin, diheptadecenoyl phosphatidylserine, pentadecenoyl sphingomyelin, heptadecanoyl lyso-phosphatidylcholine, tripheptadecaenoyl glyceride, pentadecaenoic acid, heptadecanoic cholesterol ester and free fucosterol, either individually or a combination thereof.

Separation of Lipid and Phospholipid Classes: The separation of lipid classes can be performed by preparative thin-layer chromatography (TLC), for instance using methods described herein.

To remove any residual metal or other damaging contaminants that might be on the TLC plates, each plate is washed prior to use. By way of example, the following three-step method can be used to wash the plates: impregnate each plate with ethylenediamine tetraacetic acid (EDTA), then rinse the plates once with methanol and once with chloroform. Each plate is first impregnated with 1 mM EDTA, pH 5.5, by ascending development using the method of Ruiz and Ochoa (J. Lipid Res. 38, 1482-1489, 1997). After each plate is completely developed, it was dried in air overnight. Once dry, each plate is developed in methanol, dried, and developed in chloroform, each in the same direction as the development with EDTA. The washed plates are then dried in air. Just prior to use, each plate is activated by heating it to 110 °C for 10 minutes.

To prepare the TLC chamber for chromatography, Whatman^{™} (Clifton, NJ) filter paper is cut, for instance into 20 × 80-cm strips, and wrapped around the inside wall of a glass development chamber (e.g., a chamber of 30 × 60 × 10-cm). An appropriate amount (e.g., 100 milliliters for the example container) of the desired mobile phase is added to the chamber, and the chamber sealed and allowed to equilibrate. Chambers are generally considered equilibrated when the solvent front has completely ascended the filter paper.

One representative mobile phase that can be employed for the separation of phospholipid classes is a modification of the solvent system described by Holub and Skeaf ("Nutritional regulation of cellular phosphatidylinositol," in Meth. Enzym., ed. Conn (Academic Press, Inc., Orlando), pp. 234-243, 1987) consisting of chloroform/methanol/acetic acid/water (100:67:7:4, by vol). For the separation of neutral lipid classes (total phospholipids (PL), free fatty acids (FFA), free sterols, triacylglycerides (TAG), diacylglycerides, monoacylglycerides and cholesterol esters (CE)), a solvent system consisting of petroleum ether/diethyl ether/acetic acid (80:20:1, by vol) can be used (Mangold, Thin Layer Chromatography- A Laboratory Handbook (Springer-Verlag, New York), 1969).

After the TLC plates are cooled, sample extracts are spotted onto the activated plate. In certain embodiments, samples are spotted at an estimated concentration such that no single lipid class will be present at more than 25 µg per centimeter of plate width following chromatography. This helps to ensure that the plate is not overloaded and minimized the risk of cross-contamination between lipid classes. (Cross-contamination is readily identified during sample analysis, particularly were each lipid class contains at least one unique internal standard as described herein.) Lipid class separations are performed on TLC plates, for instance with a 10-cm separation length, while PL class separations are generally performed on longer TLC plates, for instance with a 20-cm separation length.

Because lipid visualization reagents invariably degrade certain analytes, most notably the polyunsaturated fatty acids, the identification of individual lipid classes is performed by comparison with authentic lipid standards chromatographed in reference lanes. Each reference lane is spotted with a mixture of authentic lipid standards (obtained from Avanti Polar Lipids, Alabaster, AL). When the amount of sample is not limiting, the sample extract also may be spotted onto the reference lanes.

Once the TLC plates are spotted with samples and standards, and the tanks are equilibrated, the plates are transferred into the tank containing the selected mobile phase. The samples re chromatographed until the mobile phase ascended to 1-cm below the top of the plate.

Once the TLC plate is developed, the reference lipids are visualized by cutting the reference lanes from the plate, dipping the reference lanes in 10% cupric sulfate/8% phosphoric acid and charring the reference lanes at 300 °C. The charred reference lanes are used to identify the location of lipid classes on the analytical plate. In order to preserve the quantitative aspect of the sample analysis, this procedure meets the following criteria: 1) reference standards co-migrate with sample analytes with great accuracy, regardless of the source or composition of the analytes (for instance, see the mirrored control and experimental chromatograms shown in Figure 4B); 2) chromatographic separation between the lipid classes is maximized to substantially avoid cross-contamination; and 3) the portion of the plate containing analytes is not exposed to environmental stresses such as air, light or any reagent that would cause the degradation of specific analytes.

Derivatization: Once the individual lipid classes are separated, the fatty acids are hydrolyzed from their respective glycerolipids and prepared for gas chromatography. In one particular embodiment, and merely by way of example, each lipid fraction is scraped from the TLC plate using a clean razor blade and placed in a 2-mL glass vial or like container. Four-hundred microliters (400 µL) of 3N methanolic-HCl (Supelco, Bellafonte, PA) are added to each vial, and the vials are sealed under nitrogen. The sample vials are incubated at 100 °C for 45 minutes in order to trans-methylate the fatty acids. After incubation, the vials are cooled at 4 °C for 20 minutes.

The resultant fatty acid methyl esters (FAMEs) are extracted from the transmethylation-mixture with hexane. For instance, and by way of example, five-hundred microliters (500 µL) of 6% K₂CO₃ (w/v) and 200-µL of hexane, containing 0.05% butylated hydroxytoluene or another antioxidant, is added to each vial, and the vials are sealed and mixed on a vortex mixer. The sample mixture is then centrifuged at 500 × g to separate the hexane fraction, which contains the FAMEs, from the methanol/water fraction. The hexane containing the FAMEs is removed, and for instance transferred into 200-µL conical inserts and sealed in 2-mL glass tubes under nitrogen in preparation for gas chromatography. Samples may be concentrated by drying under a stream on nitrogen as necessary.

Chromatography Fatty acid methyl esters can be separated and quantified using known techniques, for instance by capillary gas chromatography using a Hewlett-Packard (Wilmington, DE) 6890 gas chromatograph. By way of non-limiting example, analysis may be performed using such a gas chromatograph equipped with a 30-m DB-225MS capillary column (J&W Scientific, Folsom, CA), and a flame-ionization detector.

Separation conditions can be determined by one of ordinary skill in the art. Representative example conditions are as follows: The injector temperature is set to 270 °C and the detector temperature set to 280 °C. The oven temperature is increased from 165 °C to 215 °C at 4.0 °C per minute and held at 215° for 12 minutes. The temperature is then increased to 230 °C at 30 °C per minute and held at that temperature for three minutes to drive off any high-boiling contaminants. Split ratios are maintained at about 40:1.

Sterols can be separated and quantified by capillary gas chromatography using a Hewlett-Packard (Wilmington, DE) 6890 gas chromatograph equipped with a 30 m DB-35MS capillary column (J&W Scientific, Folsom, CA), and a flame-ionization detector. Appropriate example separation conditions are as follows: The injector temperature is set to 310 °C and the detector temperature is set to 280 °C. The oven temperature is increased from 285 °C to 320 °C at 2.5 °C per minute. The temperature is then increased to 335 °C at 50 °C per minute to drive off any high-boiling contaminants. Split ratios are maintained at about 100:1.

The column and oven conditions may be subject to slight modification over the course of the experiment. In particular, modifications may be necessary to ensure that every fatty acid is completely resolved to baseline.

Sample chromatograms generated using the above methods are shown in Figure 4.

Optionally, a sample containing known amounts of a set of standard compounds can be run through the analysis in like fashion, to produce a control chromatogram. Such a control chromatogram is shown in Figure 4B; the constituent standard compounds are indicated.

### B. Integration and Data Handling

Following chromatography, each chromatogram is integrated, for instance using Hewlett-Packard (Wilmington, DE) ChemStation^{™} software. After chromatogram integration, the chromatogram from each sample may be visually checked to ensure proper integration. The resultant data may be sent electronically to database or spreadsheet for manipulation, for instance an Excel 2000 (Microsoft Corporation, Redmond, WA) spreadsheet. In some embodiments, the database or spreadsheet contains the sample identification information, quality control algorithms, and the algorithms required to convert the raw chromatogram data to mass or concentration data.

Appendices I and II show a single entry in an example database for control and test samples, respectively. The data structure for this specific database embodiment is discussed in more detail below.

### C. Quality Control

Several quality control protocols can be used in the described methods, to help ensure accurate, quantitative data from samples.

The rationally designed internal standards employed by the methods described herein enable true quantification of each fatty acid from each lipid class, whereas traditional lipid analysis methods produce data in either a percent-of-total format or as a mixed population of lipid metabolites. Quantitative analysis of such a mixed population of lipid classes is an analytical impossibility unless each individual class acts essentially identically at every analytical step. In addition to enabling each analysis to be highly quantitative, internal standards are designed to reflect any loss of fatty acid due to oxidation, discrimination, or cross-contamination. The results of each sample integration are analyzed by an Excel 2000 macro to determine if degradation or selective loss has occurred during the analysis. The macro automatically flags samples with standard profiles deviating by more than 2% from ideal analytical results for any fatty acid of lipid class. Flagged samples are entirely re-analyzed.

### X. Integrated Metabolomic Databases

For metabolomics to develop a global knowledge base analogous to the genome knowledge, it is imperative that data be produced and reported in quantitative terms. Typically in the past, metabolite data has been reported in a percent-of-total or other relational format. Such data have several disadvantages, including that they (1) are influenced by the number of analytes in the tested sample, (2) are influenced by co-variation between analytes, (3) are not comparable between experiments and (4) provide little basis for interpreting how metabolites interact among themselves and with other biomolecules. The quantitative data can be integrated from multiple sources (whether it is work from different labs, samples from different subjects, or merely samples processed on different days) into a single seamless database, regardless of the number of metabolites measured in each discrete analysis. Thus, abandoning rigorously quantitative methodology in return for high-throughput analyses would yield fragmented and non-integratable databases.

Further embodiments of the disclosure include databases of metabolomic data, where each database includes that metabolite quantification data from a plurality of individual lipid metabolite profiles. Such databases may be on a computer-readable storage medium, and may be formatted for processing by a computer. Data included in the databases may include any or all of the following:
information that provides for unique identification of data from a sample;
raw quantitative measurements of individual metabolites (such as lipid metabolites);
transformed measurements of individual metabolites (which have been subject to one or more mathematical transformations from raw data);
basic information about the biological sample (e.g., species, tissue, preparation date, etc.);
genetic information about the subject from which the biological sample was taken *(e.g.,* genotype of a knockout or otherwise engineered animal);
health or care history of the subject from which the sample was taken (e.g., long term care strategies, chronic conditions, etc.);
information about the treatment of the subject from which the biological sample was taken (e.g., drug application, feeding schedule or diet, stressors, environment, or toxins);
information about the harvesting of the individual sample and/or the processing of the sample;
information about the individual lipid metabolites (e.g., biochemical or biological characteristics);
information about one or more of the implicated metabolic pathways;
one or more metabolite fingerprints that are associated with a disease, condition, treatment, gene (or genotype), or drug application (*e.g*., to serve as a baseline or control sample);
information linking the treated or test samples to their experimental control samples;
information about the analytical process of producing data; and/or
information about the laboratory, investigator and analytical chemists responsible for producing the data.

The provided databases may serve to organize metabolite information, or any of the other information types indicated, in one or more tables. Such tables are readily translatable into database languages such as SQL, and the databases optionally can be integrated with an on-line Internet site containing results of user-defined metabolite analyses.

According to one aspect of the present disclosure, a computer-readable storage medium is provided, with a relational database stored on this medium. The relational database includes a metabolite table, for instance containing test metabolite data, which includes a plurality of quantitative lipid analysis records. Each record in the table includes data that corresponds to the level of a lipid metabolite in the corresponding sample.

In some embodiments, the relational database includes more than one table, for instance a control table and a test table. In some embodiments, many tables are included, for instance one each for a plurality of the different types of information described above. In some embodiments for instance, each lipid class is separated into its own table and the column headers for data are fatty acid names.

In still another embodiment the data (including additional phenotypic or biochemical data) can be stored in many related tables, with each table representing a subset of the data in its totality. For example, consider an experiment in which athletes and non-athletes are assayed for lipid metabolite profiles and resting heart rate. One format of the resultant database contains a table for each lipid class assayed by the methods described herein, with columnar data including each individual fatty acid found in each lipid class, and may also include a related table for phenotypic information, in this case resting heart rate. In this example, the results obtained from athletes and their non-athletic controls can be stored in the same table, or in a separate series of tables. The preferred embodiment would allow the two groups to be stored in the same table under unique identifying codes such that they could be queried and identified and discriminated as treatment and control from a single experiment at a later date.

Filters can be defined for sorting data in the provided databases, in order to mine the data. Examples of filter criteria based on the types of fatty acids include the following:
(1) Fatty Acid Family: In an embodiment using this filter, each fatty acid family is a filter criteria. Families may be coded by color. One representative color scheme is as follows: Black-"Saturated"; Maroon-"n7"; Blue- "n9"; Yellow- "Misc."; Green- "n3"; Red-"n6"; grey-"Trans"; Light blue- "Plasmalogen";
(2) Summary Data (summarized, for instance, by lipid family, fatty acid family, tissue, species, etc.);
(3) Major Fatty Acids Only: This filter displays only data from the following Fatty Acids: 16:0; 18:0; 16:1n7; 18:1n7; 18:1n9; 18:3n3; 20:5n3; 22:5n3; 22:6n3; 18:2n6; 18:3n6; 20:3n6; and 20:4n6.

The database format and implementation is not essential to certain elements of the disclosure. It is expected that different end users will require different systems for displaying data that are produced by the methods described herein. For instance, a specific requested display feature might dictate that the database format described herein be changed. Such modifications in database structure are known to one of ordinary skill in the art.

By way of example, one format is described below. This format is set up for speed purposes, so that the application does not need to query each value separately from the database. In this embodiment, the following information is stored for each control/treatment sample:
1) A unique auto-incrementing "id" field;
2) An integer value corresponding to the number of rows of data;
3) An integer value corresponding to the number of columns per row;
4) A string representing an identifier for the data (the name of the data); and
5) The data itself, which is stored in row-major order as a comma delimited list of values.

In this embodiment, being able to correlate two sets of data (e.g., comparing two heart tissue samples) is based on the labels matching. This database structure requires only two queries to the database before values can be computed, instead of some database formats that require on the order M*N queries, where M is the number of rows and N is the number of columns per row.

The following tables (Tables 4 and 5) present MySQL descriptions for specific embodiments:

**Table 4: mysql> describe controls;**

| **Field** | **Type** | **Null** | **Key** | **Default** | **Extra** |
|---|---|---|---|---|---|
| controlid | int(11) | | PRI | NULL | auto_increment |
| rows | int(11) | YES | | NULL | |
| cols | int(11) | YES | | NULL | |
| name | varchar(50) | YES | | NULL | |
| data | text | YES | | NULL | |

| | | | | | |
|---|---|---|---|---|---|
| 5 rows in set (0.00 sec) | | | | | |

**Table 5: mysql> describe treatments;**

| **Field** | **Type** | **Null** | **Key** | **Default** | **Extra** |
|---|---|---|---|---|---|
| treatmentid | int(11) | | PRI | NULL | auto_increment |
| rows | int(11) | YES | | NULL | |
| cols | int(11) | YES | | NULL | |
| name | varchar(50) | YES | | NULL | |
| data | text | YES | | NULL | |

| | | | | | |
|---|---|---|---|---|---|
| 5 rows in set (0.00 sec) | | | | | |

Certain embodiments of the provided databases contain at least two tables (for instance, one for controls and one for treatments), though many more tables are also contemplated.

### XI. Analysis/Mining of the Database

The database can be mined by one of many standard statistical techniques. Such techniques may include standard difference testing between or among subsets of the data selected by the user. In certain embodiments, appropriate techniques include tests such as ANOVAs, general linear models (GLM), Student's t-tests, discriminant analyses, LOGIT models, etc. For example, if a user wishes to identify any specific differences in the lipid metabolites profiles of diabetics when compared to non-diabetics, a user may select both individuals from the database that have diabetes and appropriate non-diabetic controls. To identify the lipid metabolite that best discriminates diabetics from non-diabetics; a discriminant analysis can be performed. The results of the discriminant analysis yield a single metabolite and the range of biological concentrations of that metabolite that best predicts the presence of diabetes.

A panel or profile of metabolites that predict diabetes can be created by, for instance, the following two methods, (1) by performing the described analysis repeatedly, and with each iteration, removing the discriminated metabolite or (2) by performing a discriminant analysis on summary or converted data, where the input values for the discriminant analysis are themselves values calculated from quantitative metabolite data, computed from either a random combinatorial approach or from a user-defined algorithm. A user defined algorithm can be exemplified by the following: (the sum of all fatty acids containing a delta-5 double bond) divided by (the sum of all fatty acids not containing a delta-5 double bond).

The database is mined by using a "heat map". This method of organization may include organizing the data by metabolic pathway, groupings of nutritionally related fatty acids, or the degree of difference between or among tested groups of samples.

### XII. Presentation of the Data

Presentation of data from the provided databases may be, at least in part, governed by the goal(s) of the user. Thus, it is contemplated that views and user interfaces may vary with the specific application to which the database is being put, and the specific information the user is mining from the database. The appended claims are limited to heat maps.

Heat map model: A representative example of a heat map is shown in Figure 5 In a heat map display, quantitative metabolite data from a test sample is compared to quantitative metabolite data from a base line or standard sample (a control) and the increase or decrease in each metabolite is indicated on the display, usually in a readily recognizable visual manner.

The data points can be presented in a two-dimensional layout, such as the chart shown in Figure 5, so that the columns contain data from for instance individual fatty acid chains or saturation level, while the row are arranged by lipid class, tissue type, species, or any combination thereof. Other arrangements can easily be envisioned, for instance bar graphs in two or three dimensions, which would also enable an overall picture of the data to be displayed.

By way of example, as shown in Figure 5, the increase or decrease is indicated on the display by the color of the relevant block on the chart, and the relative amount of the increase or decrease is indicated by the intensity of that color. Thus, in the embodiment pictured in Figure 5, an increase in the indicated metabolite is colored green, and the brighter the color (the further it is from black), the greater the percentage increase. Decreases may be shown in red (of varying intensity). Black can be used to indicate that there is no (or relatively little) change in the level of that metabolite. A glance at the heat map shows clearly those columns or rows that deviate from the standard, because those changes are indicated in a different color.

For instance, in the data location found in the first data column of Figure 5 (labeled 14:0), and the first row (the heart sphingomyelin (SP) sample), the test sample contained 80% more of the indicated metabolite (14:0 fatty acid, associated with sphingomyelin) than the control sample; the relevant block on the heat map is colored bright green, to indicate that the test sample had a relatively high increase in the level of this metabolite.

The number of gradations of color can be varied, depending on the sensitivity desired. The provided example displays three different intensities of red and green

Other systems than color can be used to illustrate that there is a change in the amount of a metabolite. For each such other system, a key is usually provided. By example, one non-color based system would include cross-hatching, stippling, and other "fill patterns" to indicate increases or decreases in metabolite level. In a three-dimensional depiction, the apparent height of a column (upwards or downwards from a given plain) may be indicative of the relative amount of change in the metabolite that is depicted by that column. One element of all of these embodiments (including color coding) is that patterns of change can be recognized graphically, without necessary recourse to raw or processed data numbers.

Optionally, the actual percentage increase (or decrease), or the absolute increase (or decrease) can be indicated on the heat map. In the provided example, the percentages are given for those metabolites that differ from the control sample by 10% or more (Figure 5). In alternative embodiments, the percentage can appear as a pop-up, for instance when a cursor is passed over the relevant location on the chart, or can be accessed by clicking on or otherwise indicating interest in a specific location within the chart. Relevant statistical information relating the compared data also can be presented in this way.

Data presented as a heat map can be organized in various ways, for instance, by metabolic pathway, magnitude, or direction of effect, significance of effect or by a system of categorizing the rarity or importance of an effect. An example of the importance of an effect is provided in Figure 5, which depicts many changes in lipid metabolism as the result of a pharmaceutical intervention (see Example 1). The increase in heart cardiolipin concentration is small relative to the increase in many metabolites, however, this result is rare and important to heart mitochondrial function. One benefit of organizing a heat map by tissue/organ or metabolic pathway is that it facilitates identification of systems that are strongly affected by the test condition. Similarly, other methods of organization can be used to highlight other information in the database.

In other embodiments, black is used to color the cells (locations in the heat map) representing metabolites that were not statistically different from each other. The degree of statistical significance required before coloring begins can be assigned by the user. In one embodiment, a Students t-test statistic can be calculated from the data used for comparison. The user can determine the level of significance required for coloring each cell. A standard level of significance would be a P-value of less than 0.05, which represents a 95% chance of the difference between the average of the treatment group and the control being truly different. If the difference between the average of the treatment group and the average of the control group has a P-value of less than 0.05, then the corresponding cell will be colored according to the degree of difference.

The user can define the "bin range" for the color scheme. For instance, one user may want to set a % difference of 50% to be represented by the maximum color brightness, while another user may wish to set the maximal difference to be 100%.

In some of the provided embodiments, the user is able to define the data type for display. While the database will contain quantitative data, the display type may be quantitative data (molar), quantitative (by mass), or relational by either moles or mass (mole % or weight %, respectively). These data types are easily calculated on the fly by the database engine.

The value of the differences in metabolites can be calculated in various ways, for instance as a percentage difference, a mean difference, or a percentage or mean difference of transformed data between two samples or sample groups.

### XlII. Applications

The metabolite profiles and databases produced therefrom can be used in myriad applications, including providing information about individual subjects, about disease states or other conditions, about dietary effects, about drug treatments or treatments with drug candidates, about side effects, and so forth. The provided methods and databases can be used to diagnose, prognose, and/or predict disease or other conditions, to monitor drug treatment for efficacy or side effects, to identify useful drug targets, to identify potential therapeutic agents with specific metabolic effects, or to compare the effects of multiple drugs or other compounds or conditions. Specific examples of individual applications are described more fully hereafter.

It is also contemplated that the lipid metabolomic methods and databases described herein can be used as clinical diagnostic assays, providing a comprehensive read out of lipid metabolic responses to a drug or drug treatment regimen. A clinician can use lipid metabolomic profiles, taken before, during, and after drug treatments to determine and track the effectiveness of a drug treatment. Metabolomic indicators of successful (or unsuccessful) treatment in many systems are detectable before other clinical indicators become detectable, and thus this system provides faster arid more precise characterization of an individual's response to a treatment or treatment regimen. Thus, a clinician can examine lipomic data as a way to monitor the efficacy of a particular treatment or dosing strategy, and adjust the treatment earlier than if conventional laboratory indicators are used alone.

The quantitative metabolite data, and methods for acquiring these data, provided herein can be used to identify and/or describe the complete metabolic consequences of deleting, over-expressing or otherwise changing the presence or expression of a gene. Such comparison can be used to identify the direct product of some genes, particularly those that are involved in the studied metabolic pathways (e.g., pathways of lipid catabolism or anabolism). In some embodiments, this can be used to identify the metabolic pathways affected or controlled by said gene. This type of comparison also can be used to identify what aspects of metabolism are affected by the downstream consequences of metabolic pathways controlled by the designated gene.

Quantitative lipid metabolome data as provided herein can be used as quantitative traits for gene mapping. For instance, individual fatty acid types present in single lipid classes or aggregate values, such as total number of moles of n-9 fatty acids per gram of plasma, or total moles of cardiolipin per gram of tissue, can be correlated with one or more genes. In specific embodiments, these quantitative traits are the products of an algorithm that relates metabolite values to specific genotypic changes, as the quantitative relations among metabolites are often the result of protein gene products.

Quantitative metabolite data, particularly quantitative lipid metabolite data as determined using methods described herein, can be used to identify the effects of specific pharmaceuticals, toxicological agents, or nutritional interventions (or combinations thereof) on lipid metabolism.

The methods provided herein can be used to identify one or more unknown molecular targets of a pharmaceutical, toxin or nutrient, or the metabolic function of a gene, by comparing the quantitative measurements of lipid metabolites against a quantitative database of lipid metabolites. Such a database contains the quantitative results of trials wherein the effects of genes, pharmaceuticals, toxins, or nutrients are determined and recorded. One embodiment of this approach is depicted in Figure 5.

This disclosure includes methods for comparison of the metabolic effects of two or more pharmaceutical agents, genes, toxins or nutrients by comparing the quantitative results of trials determining the quantitative effects of these compounds on lipid metabolites. Such quantitative effects can be compared by directly comparing the lipid metabolite profiles of samples that are different as regards the agent, gene, toxin, or nutrient in question.

Quantitative relationships that are defined among lipid metabolites using the methods described herein can be used to assess the relative activity or function of lipid metabolic enzymes. This approach can be used to identify protein targets of pharmaceutical agents, genes, toxins, or nutritional components.

Individualized risk assessment and directed metabolite analyses are also contemplated. The methods provided herein can be used to profile the lipid metabolites of an individual, which results are then compared to a database that contains a plurality of profiles from like and similar individuals. The individual can then be provided with, for instance, information regarding likely health risks, tendencies to disease or condition, appropriate (or inappropriate) diet, or other information garnered by comparison to the accumulated metabolomic database. In one specific embodiment, the individual profile is compared to subject that have been treated with specific drugs or who have undergone other medical treatments, and the likelihood of drug detrimental side effects for the test individual is determined. Individual analyses can be used to diagnose specific diseases or conditions that affect the metabolic system characterized by the profile and corresponding database.

Lipid metabolomics provides specific information regarding several different diseases or other conditions, including for instance organ transplant (e.g., likelihood of rejection, progress of acceptance of the donor organ), menopause (and progression through menopause), obesity, diabetes, cardiovascular disease, autoimmune conditions, responsiveness to drugs for treatment of each of these conditions (including the effectiveness of hormone therapy), and athletic performance or preparedness. Lipid metabolomic fingerprints can be prepared that provide diagnostic, predictive, and or effectiveness characteristics for each of these conditions.

### IVX. Animal Models

The methods described herein can be used to analyze animal samples and create an animal-based metabolite database, such as a lipid metabolite database, that can be mined for information.

The dominant research platform for biotechnology research is the inbred mouse. Such mice have constant genomes, making them particularly attractive as laboratory research models. They have phenotypes that mirror human diseases, and they have fixed, homozygous genomes. Because the genome of each inbred mouse strain is constant, and because the nutrition of captive research mice can be carefully controlled, phenotypic differences among strains can be attributed directly to differences in their genes.

The medical and pharmaceutical communities use these inbred mouse strains to locate and identify the genes responsible for disease and to test the efficacy of new pharmaceutical products. Although the locations and sequences of many disease-linked genes have been identified, very few of these genes have been linked with their metabolic function. Determining the metabolic function of genes is critical for validating the gene as a potential target for therapy. The methods provided herein provide the necessary link between existing genetic targets and actual metabolic function.

Lipid metabolomic profiles are produced for each inbred mouse strain under defined laboratory conditions (including, for instance, feeding and watering schedule, temperature, caging, and so forth). Profiles can be generated for a plethora of different standard condition sets. These profiles then serve as a baseline to which any modification of the strain's genome can be compared. For instance, a knockout mouse can be generated, which has been rendered defective in a single target gene. By comparing the lipid metabolite profile of the knockout mouse (or a set of such knockout mice) under defined laboratory conditions, specific metabolic effects of the gene knockout can be identified. This comparison can be used to discover, test and validate disease targets identified through genomic-, metabolomic-, or and proteomic-based techniques.

Similarly, this comparison technique can be used to examine metabolite changes caused by applying a compound to the experimental mouse(or other research animal such as monkeys), for instance by feeding the mouse the compound. Thus, drugs and drug candidates can quickly and reliably be tested for their metabolic effects.

By way of example, inbred mice strains can be selected to represent a spectrum of metabolic disease (normal growth, obesity, lean growth, and diabetes, for instance), and their baseline lipid metabolite profiles assembled into a database. This database can be queried by comparing a test lipid metabolite profile to it, and determining the similarities and differences. An animal database such as the mouse database can also be used to profile the effects of specific pharmaceutical products, for instance products that are under public scrutiny or commercial development.

In certain embodiments of the animal lipid metabolite databases, samples are assayed and lipid metabolite profiles prepared from multiple tissues from each subject mouse strain. For instance, the database may include samples from any tissue, such as one or more than one of the following: blood or blood products (such as plasma), heart, adipose (all types), liver, muscle, kidney, spleen, lung, testes, and brain.

Examples of the provided databases also may include data from different species, including for instance humans, non-human primates, and mice. Comparisons of data and data sets, as well as trends or discrepancies in metabolite levels between data from the different species, can provide identification of shared or divergent pathways between the species. Comparison of data between different species can also be used to study or predict the effects of drugs on the measured metabolites, for instance in order to predict the effects of a drug in a human system after it has been tested in an animal model.

Other specific uses for animal model databases include drug and other pharmaceutical screening, hazard models (e.g., where samples are taken from animals that have been exposed to one or more toxins, chemicals, or other hazards), and disease testing (particularly where there is a recognized model animal system that is useful for gathering comparative data that may be useful for correlation with human disease).

### VX. External Quality Control

The metabolomic databases described herein can be used to identify biological outliers in incoming data. Because certain of the provided databases contain data that defines the biological variation in each metabolite across a wide variety of species, tissues and conditions, the cumulative information base can be used to identify metabolite concentrations that are unusually high or low given prescribed criteria. These criteria can be set by the user, and may consist of restricting the data used for comparison purposes to species, tissue, treatment, age, etc.

The invention is further illustrated by the following non-limiting Examples.

### EXAMPLES

### EXAMPLE 1: Lipid metabolome-wide effects of the peroxisome proliferator-activated receptor γ agonist rosiglitazone

This example provides specific methods of generating and using quantified metabolite profiles to study the effects of a therapeutic compound.

### Samples

Mouse tissue and plasma samples were a generous donation to Lipomics Technologies from Dr. Edward Leiter of the Jackson Laboratory (Bar Harbor, ME). Samples included the plasma, heart, liver and inguinal adipose of mice treated with pharmaceuticals or their corresponding controls.

In trial 1, prediabetic male Fl mice (from a cross of the obese NZO and lean NON mouse strains) were fed a control diet with or without the presence of the PPARs-γ agonist rosiglitzazone for 4 weeks (at 0.2 g rosiglitazone per kg body weight).

In trial 2, male, inbred NZO mice were fed a control with or without the presence of the b-3 adenergenic agonist CL316,243 for four weeks (at 0.001% CL316,243 by weight in the dietary chow).

In both studies, five treated and five control mice were used. Following the treatments and the killing of the mice, tissues and plasma were taken, chilled to -80 °C and shipped to the analysis laboratory at Lipomics Technologies in a frozen state.

### Extraction

The lipids from plasma and tissues were extracted in the presence of authentic internal standards by the method of Folch et al. (J. Biol. Chem 226, 497-509, 1957) by homogenization in a fluid extractant consisting of chloroform:methanol (2:1 vol:vol). Plasma (200 µl), or 10 mg inguinal adipose tissue was used for each analysis. For each sample, an appropriate mass of internal standard was added. The internal standard compounds chosen may take many forms, but in one specific example the internal standards added to each plasma sample were: 1.75 µg of heptadecanoic 1-heptadecanoyl-2-lyso-phosphatidycholine (for lysophospholipids), 2.25 micrograms of N-pentadecenoyl-D-erythro-sphingosylphorylcholine (for sphingomyelin), 39.93 micrograms of 1,2 diheptadecanoylphosphatidylcholine (for phosphatidylcholine), 0.93 micrograms of 1,2-diheptadecenoylphosphatidylethanolamine (for phosphatidylethanolamine), 2.09 micrograms of pentadecaenoic acid (for free fatty acids), 32.93 micrograms of triheptadecaenoic acid (for triacylglycerides), 27.27 micrograms of cholesteryl heptadecanoate (for cholesterol esters) and 38.03 micrograms of stigmasterol (for free sterols).

For the analysis of liver and heart tissues, 25 mg of tissue were placed in a ground glass homogenizer and internal standards were added. The internal standards for use in the analyses of these tissues may take many forms, but in this instance consisted of: 4.75 µg of N-pentadecenoyl-D-erythro-sphingosylphorylcholine; 74.78 µg of 1,2 diheptadecanoylphosphatidylcholine; 33.57 µg of 1,2-diheptadecenoylphosphatidylserine (for phosphatidylserine); 24.13 µg of 1,2-diheptadecenoylphosphatidylethanolamine; 13.38 µg of 1,1',2,2'-tetraheptadecaenoyl cardiolipin (for cardiolipin); 1.12 µg of pentadecaenoic acid; 27.82 µg of triheptadecaenoic acid; 1.56 µg of cholesteryl heptadecanoate; and 27.70 µg of stigmasterol.

The solution mixture consisting of sample, fluid extractant, and internal standards was homogenized by twelve strokes with a ground-glass homogenizer. Following homogenization, 1.8 ml of 0.01 M potassium chloride was added, and the solution was vigorously mixed. The organic fraction containing the lipids and the internal standards was separated from the polar fraction of the mixture by centrifugation. The lipid extract was removed from the mixture and concentrated under a stream of nitrogen in preparation for lipid class separation.

### Separation of Lipid and Phospholipid Classes

The separation of lipid classes was performed by preparative thin-layer chromatography (TLC), essentially as previously described (Watkins et al., Lipids 36:247-254,2001). To remove any residual metal or other damaging contaminants on the TLC plates, each plate was washed prior to use. Washing the plates is a three-step process that involves impregnating each plate with ethylenediamine tetraacetic acid (EDTA) and rinsing the plates once with methanol and once with chloroform. Each plate is first impregnated with 1 mM EDTA, pH 5.5, by ascending development using the method of Ruiz (J. Lipid Res. 38, 1482-1489, 1997). After each plate was completely developed, it was dried in air overnight. Once dry, each plate was developed in methanol, dried, and developed in chloroform in the same direction as the development with EDTA. The washed plates were then dried in air. Just prior to use, each plate was activated by heating to 110 °C for 10 minutes.

To prepare the TLC chamber for chromatography, Whatman (Clifton, NJ) filter paper was cut into 20 × 80-cm strips and wrapped around the inside wall of a 30 × 60 × 10-cm glass development chamber. One hundred milliliters of the appropriate mobile phase was added to the chamber, and the chambers were sealed and allowed to equilibrate. Chambers were considered equilibrated when the solvent front had completely ascended the filter paper. The mobile phase employed for the separation of phospholipid classes (lyso-phospholipids, sphingomyelin, phosphatidylcholine, phosphatidylserine, phosphatidylethanolamine and cardiolipin) was a modification of the solvent system described by Holub and Skeaf ("Nutritional regulation of cellular phosphatidylinositol," in Meth. Enzym., ed. Conn (Academic Press, Inc., Orlando), pp. 234-243, 1987) consisting of chloroform/methanol/acetic acid/water (100:67:7:4, by vol).

For the separation of neutral lipid classes (free fatty acids, free sterols, triacylglycerides and cholesterol esters), a solvent system consisting of petroleum ether/diethyl ether/acetic acid (80:20:1, by vol) was used (Mangold, Thin Layer Chromatography- A Laboratory Handbook (Springer-Verlag, New York), 1969).

After the TLC plate was activated, the sample extracts were spotted onto the activated plate. As a general rule, samples were spotted at an estimated concentration such that no single lipid class was present at more than 25 µg per centimeter of plate width following chromatography. This ensured that the plate was not overloaded and minimized the risk of cross-contamination between lipid classes (cross-contamination is readily identified during sample analysis as each lipid class contains unique internal standards). Authentic lipid class standard compounds were spotted on the two outside lanes of the thin-layer chromatography plate to enable localization of the sample lipid classes.

Lipid class separations were performed on TLC plates with a 10-cm separation length, while PL class separations were performed on TLC plates with a 20-cm separation length. Because lipid visualization reagents invariably degrade certain analytes, most notably the polyunsaturated fatty acids, the identification of individual lipid classes was performed by comparison with authentic lipid standards chromatographed in reference lanes. Each reference lane was spotted with a mixture of authentic lipid standards (obtained from Avanti Polar Lipids, Alabaster, AL), and when the amount of sample is not limiting, the sample extract was also spotted onto the reference lanes. Once the TLC plates were spotted and the tanks were equilibrated, the plates were transferred into the tank containing the appropriate mobile phase, and the sample was chromatographed until the mobile phase ascended to 1-cm below the top of the plate.

Once the TLC plate is developed, the reference lipids were visualized by cutting the reference lanes from the plate, dipping the reference lanes in 10% cupric sulfate/8% phosphoric acid and charring the reference lanes at 300 °C. The charred reference lanes were used to identify the location of lipid classes on the analytical plate. Each sample was scraped from the plate using a clean razor blade and the silica scrapings were placed in a 2-mL glass vial for derivitization. Great care was taken to develop this process so that it meets the following criteria:
(1) reference standards co-migrate with sample analytes with great accuracy;
(2) chromatographic separation between the lipid classes is maximized to avoid any cross-contamination problems; and
(3) the portion of the plate containing analytes is not exposed to environmental stresses such as air, light or any reagent that would cause the degradation of specific analytes.

The silica scrapings containing the free sterol fraction were exposed to a fluid extractant consisting of one milliliter of chloroform:methanol (2:1 vol/vol). The mixture was mixed vigorously and allowed to sit for 15 minutes, then 0.3 mL of 0.01 M potassium chloride was added, and the solution once again mixed vigorously. The organic fraction containing free sterols was separated from the polar fraction of the mixture by centrifugation. The extract including free sterols was removed from the mixture and completely dried down under a stream of nitrogen. A 20-µL aliquot of chloroform was used to transfer the reconstituted free sterols to a conical insert in preparation for free sterol separation via capillary gas chromatography. No derivitization was necessary to prepare the free sterols for gas chromatographic analysis.

### Derivatization

Once the individual lipid classes were separated, the fatty acids were hydrolyzed from their respective glycerolipids and prepared for gas chromatography. Each lipid fraction was scraped from the TLC plate using a clean razor blade and placed in a 2-mL glass vial. A 400-µL aliquot of 3N methanolic-HCl (Supelco, Bellafonte, PA) was added to each vial, and the vials were sealed under nitrogen. The sample vials were incubated at 100 °C for 45 minutes in order to trans-methylate the fatty acids. After incubation, the vials were cooled at 4 °C for 20 minutes. The fatty acid methyl esters were extracted from the transmethylation-mixture with hexane. A 500-µL aliquot of 6% K₂CO₃ (w/v) and 200 µL of hexane containing 0.05% butylated hydroxytoluene as an antioxidant was added to each vial, and the vials were sealed and mixed on a vortex mixer. The sample mixture was then centrifuged at 500 × g to separate the hexane fraction, which contained the fatty acid methyl esters, from the methanol/water fraction. The hexane containing the fatty acid methyl esters was transferred into 200-µL conical inserts and sealed in 2-mL glass tubes under nitrogen in preparation for gas chromatography. Each sample was concentrated by drying the sample under a stream on nitrogen as necessary.

### Chromatography

Fatty acid methyl esters were separated and quantified by capillary gas chromatography using a Hewlett-Packard (Wilmington, DE) 6890 gas chromatograph equipped with a 30 m DB-225MS capillary column (J&W Scientific, Folsom, CA), and a flame-ionization detector, essentially as previously described (Watkins et al., Lipids 36: 247-2548, 2001). The separation conditions were as follows: The injector temperature was set to 270 °C and the detector temperature will be set to 280 °C. The oven temperature was increased from 165 °C to 215 °C at 4.0 °C per minute and held at 215 °C for 12 minutes. The temperature was then increased to 230 °C at 30 °C per minute and held at that temperature for three minutes to drive off any high-boiling contaminants. Split ratios were maintained at about 40:1. The column and oven conditions described above are subject to slight modification over the course of the experiment because this laboratory requires that every fatty acid be completely resolved to baseline for a chromatogram to pass quality control. A sample chromatogram is provided in Figure 4A.

Sterols were separated and quantified by capillary gas chromatography using a Hewlett-Packard (Wilmington, DE) 6890 gas chromatograph equipped with a 30 m DB-35MS capillary column (J&W Scientific, Folsom, CA), and a flame-ionization detector. The separation conditions were as follows: The injector temperature was set to 310 °C and the detector temperature was set to 280 °C. The oven temperature was increased from 285 °C to 320 °C at 2.5 °C per minute. The temperature was then increased to 335 °C at 50 °C per minute to drive off any high-boiling contaminants. Split ratios were maintained at about 100:1. The column and oven conditions described above were subject to slight modification over the course of the experiment because this laboratory requires that every sterol be completely resolved to baseline for a chromatogram to pass quality control.

### Integration, Data Handling and Visualization

Following chromatography, each chromatogram was integrated using Hewlett-Packard (Wilmington, DE) ChemStation^{™} software. At the beginning of each batch of samples, a standard mixture was run, containing a known concentration of each of the fatty acids listed in Table 6, below. Each fatty acid in its methyl ester form is present in this standard mixture. The quantitative standard was used to set a calibration table that automatically corrected the areas associated with each fatty acid methyl ester from the samples for injection discrimination and injector non-linearity. A representative chromatogram from a standard mixture is shown in the bottom half of Figure 4B.

Significant differences were assigned to a difference in a lipid metabolite concentration between treated and control mice on the basis of Student's t-tests (*P* ≤ 0.05).

Quantitative (nmol per g) data were visualized using the Lipomics Surveyor^{™} software system, which creates a "heat-map" graph (Figure 5) of the difference between the data for treated and control mice. The Surveyor^{™} data are read as follows: the column headers display the fatty acid and the family of fatty acids present in each lipid class, which are in turn described in the row headers. The lipid classes are grouped by tissue, and color-coded by metabolic pathway, as depicted in Figure 5. The heat map displays an increase in each metabolite in rosiglitazone-treated mice relative to control mice as a green square and a decrease in a metabolite as a red square. The brightness of the square indicates the magnitude of the difference, as detailed in the figure legends.

### RESULTS:

### Metabolomic assessment of plasma lipids

The results of the quantitative assessment of the plasma lipid metabolome in rosiglitazone-treated and untreated mice are shown in Figures 6 and 5. Lipid metabolite concentrations in plasma confirmed the rosiglitazone-induced depletion of specific classes of plasma lipids. Significant rosiglitazone-mediated decreases in phosphatidylcholine, triacylglyceride, and cholesterol ester distinguished rosiglitazone-treated mice from untreated mice, whereas no significant decreases in sphingomyelin, phosphatidylethanolamine, or free fatty acids were observed (Figure 6). Phosphatidylcholine, cholesterol ester, and triacylglycerides are derived principally from liver lipid export. Total plasma triacylglyceride concentrations were lower in treated mice (400 nmol/g) than in untreated mice (1,400 nmol/g) (Figure 6). The concentrations of total plasma free fatty acids, which are derived principally from adipose tissue, were not affected by rosiglitazone treatment. Although the total concentrations of phosphatidylcholine and cholesterol ester were lower in rosiglitazone-treated mice than in untreated mice, the absolute concentration of palmitoleic acid (16:1n7) within these lipid classes and within free fatty acids was higher in treated mice than in controls (Figure 5). The increased palmitoleic acid concentrations in plasma were reflective of the increased de novo lipogenesis occurring within the liver and adipose tissue (see below).

### Induction of de novo lipogenesis

Rosiglitazone-treated mice showed clear signs of increased *de novo* lipogenesis relative to control mice. Every lipid class in liver except sphingomyelin and the free fatty acid, cholesterol ester and total phospholipids of plasma contained a quantitative increase in palmitoleic acid (16:1n7). Additionally, the free fatty acid and triacylglycerides in adipose and every phospholipid class in heart contained an increased concentration of 16:1n7. 16:1n7 is the direct biosynthetic product of fatty acid synthase (the metabolic pathway for producing fatty acids *in vivo*) and the Δ9 desaturase. Additionally, this fatty acid was not present in the experimental diet. Hence, the substantial increase in 16:1n7 present in many liver, plasma, heart and adipose lipid classes (see Figure 5, column header "16:1n7") is the direct product of *de novo* lipogenesis.

The bright green cross-hatch pattern (horizontal- "liver TAG"; vertical- "16:1n7") combined with the clear depletion of triacylglycerides from plasma (bright red line next to "plasma TAG") visible in the "heat map" produced from the data (Figure 5) from this study suggests a dual cause for the known accumulation of lipid in the livers of rosiglitazone-treated mice. First, it is clear that the rosiglitazone treatment caused a decrease in triacylglyceride mobilization from the liver into plasma. This result is confirmed by data acquired by Dr. Edward Leiter of the Jackson Laboratory, which demonstrated an increase in the expression of genes involved in the retention of lipid by the liver. Second, the increased liver lipid content resulting from the lack of triacylglyceride mobilization is compounded by an increased *de novo* synthesis of lipid as described above.

### Liver lipid metabolism

The results of the quantitative assessment of the liver lipid metabolome in rosiglitazone-treated and untreated mice are shown in Figures 6 and 5. Lipid metabolites in the liver demonstrated a reciprocal relation between liver and plasma lipid concentrations. The significant rosiglitazone-mediated decreases in plasma triacylglycerides were balanced by a substantial accumulation of triacylglycerides within the liver (Figures 6 and 5). Total hepatic triacylglycerides were 81,300 nmol/g in untreated mice and 150,400 nmol/g in the rosiglitazone-treated mice. The concentrations of other lipid classes were not affected by rosiglitazone treatment with the exception of sphingomyelin, which was present at 1,180 nmol/g in treated mice and at 1,890 nmol/g in untreated control mice (Figure 6). This rosiglitazone-induced reciprocity between liver and plasma triacylglycerides is consistent with an inhibition of normal liver-plasma lipid exchange. No change was observed in the total concentration of phosphatidylcholine or cholesterol ester in liver as a consequence of rosiglitazone treatment (Figure 6).

### Inhibition of peroxisomal lipid metabolism

Two major types of lipids quantified in this study are derived from biosynthetic pathways present in the peroxisome. The fatty acids with three double bonds on the carboxylic acid side of an n-9 double bond (22:5n6 and 22:6n3) are synthesized by retroconversion from their biosynthetic precursors (24:5n6 and 24:6n3, respectively) in the peroxisome. The plasmalogen lipids, those lipids that contain one or more 1-enyl-ether-linked fatty acids, are also derived from biosynthetic pathways present in the peroxisome. In hearts from mice treated with rosiglitazone there was a substantial decrease in the 22:6n3 content of all phospholipid classes except sphingomyelin, as well as in free fatty acids and cholesterol esters, relative to control mice. Additionally, there was a significant depletion of 1-enyl-ether linked fatty acids from the heart phospholipids of rosiglitazone-treated mice relative to control mice. These observations are easily detectable in Figure 5, which portrays this data in the described "heat map" format. Each of these observations suggests that rosiglitazone, a known PPARs-γ agonist, has an inhibitory effect on lipid synthesis in the peroxisome.

### Heart lipid class metabolism

The results of the quantitative assessment of the heart lipid metabolome in rosiglitazone-treated and untreated mice are shown in Figures 7 and 5. Free fatty acids are the primary source of energy for the heart. The average concentration of total free fatty acids in the heart was 5,100 nmol/gin untreated mice and 2,500 nmol/g in rosiglitazone-treated mice (Figure 6). This difference was largely independent of the type of free fatty acid, as the saturated n-3, n-6, and n-9 families of fatty acids were all approximately 50% lower in treated mice than in untreated mice (Figure 6). The free n-7 fatty acids were not depleted as substantially from heart, likely due to the increased biosynthesis of n-7 fatty acids and corresponding increased concentration of n-7 fatty acids within the triacylglycerides and free fatty acids of plasma.

The hearts of rosiglitazone-treated mice were significantly enriched with cardiolipin, the primary structural lipid of the inner mitochondrial membrane. The mean cardiolipin content of hearts from rosiglitazone-treated mice was 3,000 nmol/g as compared with 2,500 nmol/g in untreated mice. Unlike free fatty acids, the fatty acid components of cardiolipin were differentially modulated by rosiglitazone treatment. The primary fatty acid of cardiolipin, linoleic acid (18:2n6), was 4,550 nmol/g in control heart cardiolipin and 8,850 nmol/g in heart cardiolipin of rosiglitazone-treated mice. Docosahexaenoic acid (22:6n3) was depleted from cardiolipin in the hearts of treated mice (950 nmol/g) relative to hearts of control mice (2,200 nmol/g).

The plasmalogen lipids, those lipids that contain 1-enyl-ether-linked alkyl chains, are derived from the dihydroxyacetone phosphate pathway and are partially synthesized within the peroxisome. The concentration of plasmalogens was lower in the heart phospholipids of mice treated with rosiglitazone than of controls (Figure 5). These data are consistent with a decreased peroxisomal synthesis of lipids within the hearts of treated mice.

### Adipose lipid class metabolism

The results of the quantitative assessment of the inguinal adipose lipid metabolome in rosiglitazone-treated and untreated mice are shown in Figures 6 and 5. Inguinal fat tissue from rosiglitazone-treated mice displayed a 5.7% lower triacylglyceride content (9,628 µmol/g) than inguinal adipose from controls (1,019 µmol/g), and 35% more free fatty acids (13,370 nmol/g in treated mice and 9,900 nmol/g in controls). No significant differences in total phospholipid or cholesterol ester concentrations were observed (Figure 6).

The fatty acid composition of inguinal fat triacylglycerides was substantially altered by rosiglitazone treatment, with inguinal fat from treated mice accumulating fatty acids from the saturated n-7 and n-3 families of fatty acids, while being depleted of the n-9 family of fatty acids (Figure 6). In particular, an unusual accumulation of n-3 fatty acids was observed in inguinal fat from rosiglitazone-treated animals. The concentration of total n-3 fatty acids in the inguinal fat triacylglycerides of treated mice was 71,260 nmol/g, representing a 120% greater concentration than that in untreated mice (Figure 6). The most notable increases within the n-3 family of fatty acids were a 522% greater concentration (4,100 nmol/g) of eicosapentaenoic acid, a 612% greater concentration (7,000 nmol/g) of docosahexaenoic acid, and 84% (24,300 nmol/g) more α-linolenic acid in inguinal fat triacylglycerides in treated as compared with control mice (Figure 5). The concentration of n-7 fatty acids in inguinal fat triacylglycerides was 303 µmol/g in treated mice and 204 µmol/g in untreated controls (Figure 6). In contrast, the total concentration of n-6 fatty acids was less than 3% higher. However, the accumulation or depletion of individual fatty acids within the n-6 family varied substantially. Whereas linoleic acid (18:2n6), by far the most prominent n-6 fatty acid in inguinal fat, was not significantly altered by treatment, the concentrations of γ-linolenic, dihomo-γ-linolenic, and arachidonic acids in inguinal fat were respectively, 1,225 nmol/g (78 %), 1,300 nmol/g (64 %), and 3,800 nmol/g (276 %) greater in treated mice than in untreated controls (Figure 5).

The concentration of plasmalogen lipids in inguinal fat phospholipids was depleted by rosiglitazone treatment (Figure 6). The concentration of total plasmalogen fatty acids from the phospholipids of inguinal fat was 130 nmol/g (60%) less in treated mice than untreated controls.

### Differential effects on individual organs

It is clear from Figure 5 that the effect of rosiglitazone is variable on different tissues, and that a complete metabolomic assessment, including the measurement of both fatty acids and lipid classes from several tissues is important for understanding the true effects of rosiglitazone on lipid metabolism.

### Discussion

Rosiglitazone treatment is often accompanied by weight gain in humans, an effect strikingly reflected by the rosiglitazone-induced increase in body weight of already markedly-obese (NZO x NON)F1 male mice. In this study, the potent anti-hyperglycemic effect of rosiglitazone was accompanied by an increased *de novo* synthesis of fatty acids. Palmitoleic acid (16:1n7) and vaccenic acid (l8:1n7) were excellent metabolic indicators of the increased *de novo* synthesis of fatty acids, and the effect appeared to be mediated by an increased expression of fatty acid synthase within in the liver. This increased synthesis of fatty acids is likely a key metabolic explanation for both the weight gain and the severe hepatic steatosis observed in the rosiglitazone-treated animals. Interestingly, although lipid biosynthesis was increased, the increase in liver triacylglyceride concentration was not reflected in the plasma. Thus, there is a strong indication that normal lipid import-export activities between the liver and plasma were impaired by rosiglitazone treatment, and that this dysregulation and increased biosynthesis of lipids may be mutually responsible for the **hepatic steatosis.**

Because rosiglitazone decreased the concentrations of plasma lipids as classes of molecules (i.e., triacylglycerides, cholesterol esters, etc.), standard clinical markers of lipid metabolism did not reflect the increased hepatic *de novo* lipogenesis in response to rosiglitazone treatment. In contrast, the metabolomic assessment of plasma lipids identified several markers of increased liver lipogenesis, including an increased absolute concentration of 16:1n7 and 18:1n7 in plasma cholesteryl esters, phosphatidylcholine, and triacylglycerides, despite the decrease in the concentration of total plasma lipid classes. The metabolomic analysis of the plasma alone was therefore capable of making the important discrimination between hypolipidemia caused by decreased lipid synthesis compared with hypolipidemia caused by impaired export of lipid by the liver. These data suggest that metabolomic analyses of human plasma have strong potential as clinical diagnostics. Further demonstrating the strong relations between the plasma lipid metabolome and tissue metabolism were the decreased concentration of plasmalogen lipids in plasma and the similarity between the composition of the plasma lipid metabolome and liver and adipose metabolomes.

Heart lipid metabolism was strongly influenced by rosiglitazone treatment. In particular, heart free fatty acids, cardiolipin, plasmalogen lipids, and the important polyunsaturated fatty acids 22:6n3 and 18:2n6 were significantly modulated by treatment. Some of these changes, particularly those involving the concentration and composition of cardiolipin and free fatty acids, may in part represent the alterations in muscle metabolism that improve insulin sensitivity. Cardiolipin is an essential phospholipid for energy metabolism and the primary phospholipid of the inner mitochondrial membrane. The content and composition of cardiolipin are important to the efficiency of electron transport. Rosiglitazone caused an increase in heart cardiolipin concentration and a substantial remodeling of cardiolipin toward an elevated 18:2n6 content and a diminished 22:6n3 content. Interestingly, this is precisely the change in cardiolipin content and composition that would increase electron transport efficiency and decrease electron leakage, according to the existing *in vitro* data. Rosiglitazone-induced remission from hyperglycemia in combination with reduced plasma insulin concentrations indicated that glucose oxidation by tissues was increased by this insulin-sensitizing agent. Thus, it is possible that increased energy metabolism as well as decreased plasma lipids may have caused the decreased heart free fatty acid concentrations.

Two major types of lipids quantified in this study are synthesized at least in part within the peroxisome. These are the fatty acids with three double bonds on the carboxylic acid side of an n9 double bond (22:5n6 and 22:6n3) (Moore et al., J. Lipid Res., 36:2433-2443, 1995; Sprecher et al., J. Lipid Res., 36:2471-2477, 1995; Voss et al., J. Biol. Chem. 266:19995-20000, 1991), and the plasmalogen lipids, which are synthesized by the dihydroxyacetone phosphate biosynthetic pathway (Nagan & Zoeller, Prob. in Lipid Res., 40:199-229, 2001). Heart tissue from rosiglitazone-treated mice contained significantly less 22:6n3 in phosphatidylcholine, phosphatidylethanolamine, cardiolipin, phosphatidylserine/inositol, free fatty acids, and cholesterol esters than did heart from untreated control mice. Additionally, there was a significant depletion of plasmalogen lipids from the heart phospholipids of treated mice relative to untreated controls. These observations suggest that rosiglitazone, a known PPARγ agonist, has an inhibitory effect on lipid biosynthesis in the peroxisome. The decreased production of 22:6n3 and plasmalogen lipid may have important physiologic consequences. Dietary 22:6n3 has well-documented positive effects on cardiac function, and plasmalogen lipids have recently been shown to be essential to membrane trafficking and the structure of caveolae, clathrin-coated pits, endoplasmic reticulum, and Golgi cisternae.

A curious finding in this study was the inguinal fat tissue accumulation of polyunsaturated fatty acids in response to rosiglitazone. Accumulation of 22:6n3 and other long-chain polyunsaturated fatty acids likely occurs via a pathway independent of their biosynthesis *de novo* from precursors. The conversion of polyunsaturated-rich phospholipids to triacylglycerides via a phospholipase D pathway also does not appear to be the primary metabolic basis for the enrichment with polyunsaturates, as phospholipids were also enriched with polyunsaturated fatty acids. This unusual response may be an important clue to understanding the physiology of adipose tissue activated by PPARγ agonists, and should be investigated further.

The present study utilized a diabetic mouse model in which the anti-diabetic action of a TZD was accompanied by excessive weight gain and major alterations in the lipid metabolome. Its major findings were that rosiglitazone (*i*) induced hypolipidemia by disrupting the mobilization of liver lipids into plasma, (*ii*) induced *de novo* fatty acid synthesis, (*iii*) diminished the biosynthesis of lipid synthesized within the peroxisome, (iv) had substantial effects on heart cardiolipin and free fatty acid metabolism, and (v) exerted tissue-specific effects on lipid metabolism.

The results presented above clearly demonstrate that metabolomic data can be obtained, stored, visualized, and analyzed using methods provided herein.

### EXAMPLE 2: Disease/Condition-Linked Lipid Metabolite Profiles (Fingerprints)

With the provision herein of methods for determining the quantitative levels of a comprehensive panel of lipid metabolites, and the ability to assemble such individual metabolite profiles into a minable database, disease- or condition-linked lipid metabolite profiles (which provide information on the disease or condition state of a subject) are now enabled.

Disease or condition linked lipid metabolite profiles comprise the distinct and identifiable pattern of levels of lipid metabolites, for instance a pattern of high and low levels of a defined set of metabolites or subset of like or unlike metabolites, or molecules that can be correlated to such metabolites (such as biosynthetic or degradative enzymes that affect such metabolites). The set of molecules in a particular profile usually will include at least one of those listed in Table 6.

**Table 6:**

| **SCIENTIFIC NAME** | **SCIENTIFIC ABBR.** | **COMMON NAME** |
|---|---|---|
| **- SATURATED -** | | |
| Tetradecanoic Acid | 14:0 | Myristic Acid |
| Pentadecanoic Acid | 15:0 | - |
| Hexadecanoic Acid | 16:0 | Palmitic Acid |
| Heptadecanoic Acid | 17:0 | Margaric Acid |
| Octadecanoic Acid | 18:0 | Stearic Acid |
| Eicosanoic Acid | 20:0 | Arachidic Acid |
| Docosanoic Acid | 22:0 | Behenic Acid |
| Tetracosanoic Acid | 24:0 | Lignoceric Acid |
| - **D9 DESATURASE FAMILY -** | | |
| 9-Tetradecenoic Acid | 14:1n5 | Myristoleic Acid |
| 9-Hexadecenoic Acid | 16:1n7 | Palmitoleic Acid |
| 11-Octadecenoic Acid | 18:1n7 | Vaccenic Acid |
| 9-Octadecenoic Acid | 18:1n9 | Oleic Acid |
| 11-Eicosenoic Acid | 20:1n9 | Eicosenoic Acid |
| 5,8,11-Eicosatrienoic Acid | 20:3n9 | Mead Acid |
| 13-Docosenoic Acid | 22:1n9 | Erucic Acid |
| 15-Tetracosenoic Acid | 24: 1 n9 | Nervonic Acid |

| **- OMEGA 3 FAMILY -** | | |
|---|---|---|
| 9,12,15-Octadecatrienoic Acid | 18:3n3 | a-Linolenic Acid |
| 6,9,12,15-Octadecatetraenoic Acid | 18:4n3 | - |
| 11,14,17-Eicosatrienoic Acid | 20:3n3 | Eicosatrienoic Acid (ETA) |
| 8,11,14,17-Eicosictetraenoic Acid | 20:4n3 | - |
| 5,8,11,14,17-Eicosapentaenoic Acid | 20:5n3 | Eicosapentaenoic Acid (EPA) |
| 7,10,13,16,19-Docosapentaenoic Acid | 22:5n3 | Docosapentaenoic Acid (DPA) |
| 4,7,10,13,16,19-Docosahexaenoic Acid | 22:6n3 | Docosahexaenoic Acid (DHA) |
| 6,9,12,15,18,21-Tetracoshexaenoic Acid | 24:6n3 | Tetracosahexaenoic Acid |
| **- OMEGA 6 FAMILY -** | | |
| 9,12-Octadecadienoic Acid | 18:2n6 | Linoleic Acid |
| 6,9,12-Octadecatrienoic Acid | 18:3n6 | g-Linolenic Acid |
| 11,14-Eicosadienoic Acid | 20:2n6 | Eicosadienoic Acid |
| 8,11,14-Eicosatrienoic Acid | 20:3n6 | Homo-g-Linolenic Acid |
| 5,8,11,14-Eicosicatetraenoic Acid | 20:4n6 | Arachidonic Acid |
| 13,16-Docsadienoic Acid | 22:2n6 | Docosadienoic Acid |
| 7,10,13,16-Docosicatetraenoic Acid | 22:4n6 | Docosicatetraenoic Acid |
| 4,7,10,13,16-Docosapentaenoic Acid | 22:5n6 | Docosapentaenoic Acid |
| **- UNUSUAL FAMEs -** | | |
| 9-Trans-Hexadecenoic Acid | t16:1n7 | Palmitelaidic Acid |
| 9-Trans-Octadecenoic Acid | t18:1n9 | Elaidic Acid |
| 8-Eicosaenoic Acid | 20:1n12 | - |
| 5-Eicosaenoic Acid | 20:1n15 | - |
| Plasmalogen fatty acids | 16:0 | - |
| " | 18:0 | - |
| " | 18:1n7 | - |
| " | 18:1n9 | - |
| **-STEROLS-** | | |
| 5b-cholestan-3b-ol | C₂₇H₄₈O | coprostanol |
| 5a-cholestan-3b-ol | C₂₇H₄₈O | dihydrocholesterol |
| 5-cholesten-3b-ol | C₂₇H₄₆O | cholesterol |
| 5,24-cholestadien-3b-ol | C₂₇H₄₄O | desmosterol |
| 5-cholestan-25a-methyl-3b-ol | C₂₈H₄₂O | campesterol |
| 5-cholestan-24b-methyl-3b-ol | C₂₈H₄₂O | dihydrobrassicasterol |
| 5-cholesten-24b-ethyl-3b-ol | C₂₉H₅₀O | b-sitosterol |
| 5,22-cholestadien-24b-ethyl-3b-ol | C₁₉H₄₈O | stigmasterol |

By way of example, any subset of the metabolites listed in Table 6 may be included in a single lipid metabolite profile. Specific examples of such subsets include those metabolites (1) that are linked by a biosynthetic or biodegradative pathway, (2) that are precursors or products of each other, and so forth. Alternatively, some subsets include those metabolites that show an increasing level during progression of a disease or condition such as diabetes, obesity, heart disease, coronary artery disease, liver disease, menopause, pregnancy, or hyper- or hypothyroidism; those that show a decreasing level; those that are most highly correlated to a particular stage or progression of a specified disease or condition, and so forth. Alternatively, lipid metabolite profiles may be further broken down by the tissue from which metabolites were harvested for the profile. Thus, certain examples of profiles may include a specific class of lipid metabolites that are found only in, or are found only to be affected in, a specific tissue, such as heart, nerve (such as brain), liver, adipose, connective, or other tissue. In some instances, selection of such tissue-specific profiles may be guided by existing knowledge that that tissue (or those tissues) is involved in the disease or condition under study.

Particular metabolite profiles are specific for a particular stage of normal tissue (*e*.*g*., normal heart tissue), a particular nutritional state (e.g., growth on a particular diet), a particular condition or disease (e.g., diabetes), or a disease or condition progression (e.g., progression of menopause, for instance as a set of profiles from a single subject over a period of time prior to, during, and after onset of menopause). Each profile includes information on the level of a set of lipid metabolites that are linked to the disease or condition being studied (*e*.*g*., menopause-progression linked metabolites). Such information usually includes absolute levels of specific metabolites, and may similarly include the levels of a class (or classes) of metabolites that are linked by a biochemical pathway, or metabolites that are otherwise biochemically related to each other. Results from the lipid metabolite profiles of an individual subject are often viewed in the context of a test sample compared to a baseline or control sample profile, or a known profile compiled from a database of individual profiles.

The levels of lipid metabolites that make up a lipid metabolite profile can be measured in any of various known ways, including specifically those methods described herein. In particular, it is contemplated that any method that can be used to generate a quantitative measurement of individual metabolites, particularly a chromatographic method, can be used to generate data for use in the described lipid metabolite profiles.

### EXAMPLE 3: Identification of Compounds

The linkage of specific lipid metabolites, or sets of lipid metabolites, and the levels thereof (for instance, as shown in a lipid metabolite profile), to a disease, condition, or predilection of an individual to suffer from or progress in a disease or condition, can be used to identify compounds that are useful in treating, reducing, or preventing that disease or condition, or development or progression of the disease or condition.

By way of example, a test compound is applied to a cell, for instance a test cell, and a lipid metabolite profile is generated and compared to the equivalent measurements from a test cell that was not so treated (or from the same cell prior to application of the test compound). Similarly, in some embodiments, the test compound is applied to a test organism, such as a mouse. If application of the compound alters level(s) of one or more lipid metabolites (for instance by increasing or decreasing that level), or changes the lipid metabolite profile, then that compound is selected as a candidate for further characterization.

Control lipid metabolite profiles useful for comparison in such methods may be constructed from, for instance, normal tissue or cells, tissue or cells taken from a subject known to suffer from the target disease/condition or a specific stage of that disease/condition, tissue or cells that have been or are being subject to a treatment for that disease or condition, and/or a tissue or cells taken from a subject known to suffer from a different disease/condition or stage thereof. In the latter example, the different disease/condition may be a disease or condition that is known to affect a similar set or subset of lipid metabolites, known to be influenced by similar drugs or treatments, or is not related to the target disease/condition with any currently identified correlation.

## Claims

1. A method for analyzing a plurality of quantitative lipid metabolite profiles, also known as lipomic profiles, comprising:
(a) generating a quantitative lipid metabolite profile, comprising:
(i) separating a biological sample into fractions based on a plurality of lipid classes, wherein at least one quantitative internal standard is included for each lipid class; and
(ii) measuring the quantity of a plurality of lipid metabolites in each of the fractions, wherein the quantitative measurements of the plurality of lipid metabolites in the fractions are stored as the quantitative lipid metabolite profile;
(b) designating a plurality of such quantitative lipid metabolite profiles;
(c) identifying a plurality of differences or similarities in the measured lipid metabolites between the plurality of designated quantitative lipid metabolite profiles; and
(d) displaying the plurality of differences or similarities in the lipid metabolites between the plurality of quantitative lipid metabolite profiles, wherein the displaying comprises displaying a heat map, so as to enable the user an easy comparison between the plurality of lipid metabolite profiles.

2. The method of claim 1, which further comprises
generating each of the plurality of quantitative lipid metabolite profiles designated in (b), wherein the generation of each of the quantitative lipid metabolite profiles comprises:
(i) separating a biological sample into fractions each corresponding to one of a plurality of lipid classes, wherein at least one quantitative internal standard is included for each lipid class; and
(ii) measuring the quantity of a plurality of lipid metabolites in each of the fractions, wherein the quantitative measurements of the plurality of lipid metabolites in the fractions are stored as the quantitative, lipid metabolite profile.

3. The method of claim 1 or 2, wherein the plurality of lipid classes are classes which comprise lyso-phosphatidylcholines, sphingomyelins, phosphatidylcholines, phosphatidylserines, phosphatidylinositols, phosphatidylethanolamines, cardiolipins, free fatty acids, monoacylglycerides, diacylglycerides, triacylglycerides, and/or cholesterol esters.

4. The method of claim 1 or 2, which further comprises
assembling the plurality of lipomic profiles into a database.

5. The method of claim 1 or 2, wherein the lipid metabolites are selected from the group consisting of tetradecanoic acid, pentadecanoic acid, hexadecanoic acid, heptadecanoic acid, octadecanoic acid, eicosanoic acid, docosanoic acid, tetracosanoic acid, 9-tetradecenoic acid, 9-hexadecenoic acid, 11-octadecenoic acid, 9-octadecenoic acid, 11-eicosenoic acid, 5,8,11-eicosatrienoic acid, 13-docosenoic acid, 15-tetracosenoic acid, 9,12,15-octadecatrienoic acid, 6,9,12,15-octadecatetraenoic acid, 11,14,17-eicosatrienoic acid, 8,11,14,17-eicosictetraenoic acid, 5,8,11,14,17-eicosapentaenoic acid, 7,10,13,16,19-docosapentaenoic acid, 4,7,10,13,16,19-docosahexaenoic acid, 6,9,12,15,18,21-tetracoshexaenoic acid, 9,12-octadecadienoic acid, 6,9,12-octadecatrienoic acid, 11,14-eicosadienoic acid, 8,11,14-eicosatrienoic acid, 5,8,11,14-eicosicatetraenoic acid, 13,16-docsadienoic acid, 7,10,13,16-docosicatetraenoic acid, 4,7,10,13,16-docosapentaenoic acid, 9-trans-hexadecenoic acid, 9-trans-octadecenoic acid, 8-eicosaenoic acid, 5-eicosaenoic acid, plasmalogen fatty acids, 5b-cholestan-3b-ol, 5a-cholestan-3b-ol, 5-cholesten-3b-ol, 5,24-cholestadien-3b-ol, 5-cholestan-25a-methyl-3b-ol, 5-cholestan-24b-methyl-3b-ol, 5-cholesten-24b-ethyl-3b-ol, and 5,22-cholestadien-24b-ethyl-3b-ol, each as a compound or a component of a lipid molecule.

6. The method of claim 1 or 2, wherein the lipid metabolites are selected from the group consisting of fatty acid 16:0, 18:0, l6:1n7; 18:1n7; 18:1n9; 18:3n3; 20:5n3; 22:5n3; 22:6n3; 18:2n6; 18:3n6; 20:3n6; and 20:4n6, each as a compound or a component of a lipid molecule.

7. The method of claim 1 or 2, wherein the lipid metabolites are selected from the group consisting of 5b-cholestan-3b-ol, 5a-cholestan-3b-ol, 5-cholesten-3b-ol, 5,24-cholestadien-3b-ol, 5-cholestan-25a-methyl-3b-ol, 5-cholestan-24b-methyl-3b-ol, 5-cholesten-24b-ethyl-3b-ol, and 5,22-cholestadien-24b-ethyl-3b-ol, each as a compounds or a component of a lipid molecule.

8. The method of claim 1 or 2, wherein the internal standard is selected from the group consisting of diheptadecanoyl phosphatidylcholine, dipentadecaenoyl phosphatidylethanolamine, tetraheptadecenoyl cardiolipin, diheptadecenoyl phosphatidylserine, pentadecenoyl sphingomyelin, heptadecanoyl lyso-phosphatidylcholine, tripheptadecaenoyl glyceride, pentadecaenoic acid, heptadecanoic cholesterol ester and free fucosterol.

9. The method of claim 1 or 2, wherein the internal standard is heptadecanoic 1-heptadecanoyl-2-lyso-phosphatidycholine for the lipid class of lysophospholipids, N-pentadecenoyl-D-erythro-sphingosylphorylcholine for the lipid class of sphingomyelin, 1,2 diheptadecanoylphosphatidylcholine for the lipid class of phosphatidylcholine, 1,2-diheptadecenoylphosphatidylethanolamine for the lipid class of phosphatidylethanolamine, 1,2-diheptadecenoylphosphatidylserine for the lipid class of phosphatidylserine, pentadecaenoic acid for the lipid class of free fatty acids, triheptadecaenoic acid for the lipid class of triacylglycerides, 1,1',2,2'-tetraheptadecaenoyl cardiolipin for the lipid class of cardiolipin, cholesteryl heptadecanoate for the lipid class of cholesterol esters and stigmasterol for the lipid class of free sterols.

10. The method of any preceding claim, wherein separating comprises chromatography and/or measuring comprises chromatography.

11. The method of claim 1 or 2, wherein displaying generates a web page for viewing.

12. A method of determining a metabolic effect of a condition, comprising
analyzing a plurality of quantitative lipomic profiles according to claim 1, wherein the plurality of quantitative lipomic profiles comprises a quantitative lipomic profile from a subject that has been subjected to the condition and a quantitative control lipomic profile; and
drawing conclusions about the metabolic effect of the condition based on differences or similarities between the quantitative lipomic profile from the subject and the quantitative control lipomic profile.

13. The method of claim 12, wherein the condition comprises:
a genotype;
a non-human genetic knockout;
dietary limitation or supplementation;
a disease or disease state;
application of a toxin or suspected toxin; or
application of a pharmaceutical agent or candidate agent.

14. The method of claim 12, wherein the control lipomic profile is:
a compiled lipomic profile assembled from a plurality of individual lipomic profiles; or
a pre-condition lipomic profile from the subject.

15. The method of claim 12, wherein the condition comprises a hormone or hormone treatment or influences obesity or diabetes.

16. The method of any one of claims 1 through 16, further comprising generating a printed report.

17. The method of claim 4, wherein the database comprises:
(1) a profile table including a quantitative lipid metabolic profile from a biological sample from an individual having a condition, wherein the quantitative lipid metabolic profile comprises a quantified measurement of a lipid metabolite and wherein the quantified measurement is obtained using an internal standard for the lipid metabolite so that the quantified measurement is integratable into a database;
(2) a sample item table including a sample record for the quantitative lipid metabolic profile;
(3) a condition item table including a condition record for the quantitative lipid metabolic profile; and
(4) a filter item table including a filter of the quantitative lipid metabolic profile for a desired condition.

18. The method of claim 4, further comprising a user interface for operatively working with a processor to affect operation of the database, wherein the user interface comprises:
means for providing settings for selecting a set of samples,
means for providing settings for selecting a set of conditions,
means for providing settings for selecting a set of lipid metabolites, and
means for displaying quantitative lipid metabolic profiles corresponding to the selected samples and conditions, wherein each displayed quantitative lipid metabolic profile consists of the quantified measurements of the selected lipid metabolites.

19. The method of claim 18, wherein the user interface further comprises a display area which displays the value of a quantified measurement of a lipid metabolite within the quantitative lipid metabolic profiles of the selected samples and conditions.

20. The method of claim 18, wherein the user interface further comprises
means for comparing quantitative lipid metabolic profiles corresponding to a first set of selected samples and conditions to the quantitative lipid metabolic profiles corresponding to a second set of selected samples and conditions, and
means for displaying the comparison.

21. The method of claim 18, wherein the user interface comprises:
for a plurality of lipid metabolites, a presentation of an observed quantity of at least one lipid metabolite for a first biological sample with respect to an observed quantity of the at least one lipid metabolite for a second biological sample, wherein the presentation is operable to accept a user indication that further information is desired with respect to a selected lipid metabolite.

22. The method of claim 1 or 2, wherein each of the quantitative measurements of the plurality of lipid metabolites in the fractions stored as the quantitative metabolite profile is stored as a mass or concentration measurement.

23. The method of claim 1 or 2, wherein the quantities of more than 35 individual fatty acids from each of the lipid classes are measured.

24. The method of claim 1 or 2, wherein the plurality of lipid classes comprises a lipid class selected from the group consisting of phosphatidylcholines, phosphatidylserines, phosphatidylinositols, phosphatidylethanolamines, and cardiolipin.

25. The method of claim 1 or 2, wherein an increase or decrease of a measured lipid metabolite is indicated on the heat map display by the color and the relevant amount of the increase or decrease is indicated by the intensity of that color.

26. The method of claim 1 or 2, wherein the plurality of lipid classes comprises each of phospholipids, glycerides, and other lipids.

## Patentansprüche

1. Verfahren zum Analysieren einer Vielzahl von quantitativen Lipidstoffwechselprodukt-Profilen, die auch als Lipoprofile bekannt sind,
**gekennzeichnet durch** folgende Schritte;
(a) ein quantitatives Lipidstoffwechselprodukt-Profil wird in folgenden Schritten erzeugt:
(i) Trennen eines biologischen Musters in Fraktionen, die auf einer Vielzahl von Lipidklassen basieren, wobei mindestens ein quantitativer innerer Standard für jede Lipidklasse eingeschlossen ist, und
(ii) Messen der Menge einer Vielzahl von Lipidstoffwechselprodukten in jeder der Fraktionen, wobei die quantitativen Messungen der Vielzahl der Lipidstoffwechselprodukte in den Fraktionen als quantitative Lipidstoffwechselprodukt-Profile gespeichert werden,
(b) eine Vielzahl dieser quantitativen Lipidstoffwechselprodukt-Profile wird bestimmt,
(c) eine Vielzahl von Unterschieden oder Ähnlichkeiten in den gemessenen Lipidstoffwechselprodukten unter der Vielzahl von bestimmten, quantitativen Lipidstoffwechselprodukt-Profilen wird identifiziert und
(d) die Vielzahl von Unterschieden oder Ähnlichkeiten in den LipidstoffwechselProdukt-Profilen unter der Vielzahl der quantitativen Lipidstoffwechselprodukt-Profilen wird angezeigt, wobei diese Anzeige das Anzeigen einer Laufkarte umfasst, derart, dass der Benutzer die Lipidstoffwechselprodukt-Profile leicht miteinander vergleichen kann.

2. Verfahren nach Anspruch 1,
**dadurch gekennzeichnet,**
**dass** es ferner folgenden Schritt umfasst:
jedes der Vielzahl von im Schritt (b) bestimmten, quantitativen Lipidstoffwechselprodukt-Profilen wird erzeugt, wobei die Erzeugung jedes dieser Lipidstoffwechselprodukt-Proflle folgende Schritte umfasst:
(i) ein biologisches Muster wird in Fraktionen getrennt, die jeweils zu einer Vielzahl von Lipidklassen gehören, wobei mindestens ein quantitativer, innerer Standard für jede Lipidklasse vorgesehen ist, und
(ii) die Menge einer Vielzahl von Lipidstoffwechselprodukten in jeder der Fraktionen wird gemessen, wobei die quantitativen Messungen der Vielzahl von Lipidstoffwechselprodukten in den Fraktionen als quantitative Lipidstoffwechselprodukt-Profile gespeichert werden.

3. Verfahren nach Anspruch 1 oder 2,
**dadurch gekennzeichnet,**
**dass** die Vielzahl der Lipidklassen Klassen sind, die Lysophosphatidylcholine, Sphingomyeline, Phosphatidylcholine, Phosphatidylserine, Phosphatidylinositole, Phosphatidylethanolamine, Kardiolipine, freie Fettsäuren, Monoacylglyceride, Diacylglyceride, Triacylglyceride und/oder Cholesterolester aufweisen.

4. Verfahren nach Anspruch 1 oder 2,
**dadurch gekennzeichnet,**
**dass** es ferner den Schritt der Aufnahme der Vielzahl der Lipoprofile in einer Datenbank umfasst.

5. Verfahren nach Anspruch 1 oder 2,
**dadurch gekennzeichnet,**
**dass** die Lipidstoffwechselprodukte aus der Gruppe ausgewählt sind, die Tetradekansäure, Pentadekansäure, Hexadekansäure, Heptadekansäure, Oktadekansäure, Eikosansäure, Dekosansäure, Tetrakosansäure, 9-Tetradekensäure, 9-Hexadekensäure, 11-Oktadekensäure, 9-Oktadekensäure, 11-Eikosensäure, 5,8,11-Eikosatriensäure, 13-Dokosensäure, 15-Tetrakosensäure, 9,12,15-Oktadekatriensäure, 6,9,12,15-Oktadekatetraensäure, 11,14,17-Eikosatriensäure, 8,11,14,17-Eikosiktetraensäure, 5,8,11,14,17-Eikosapentaensäure, 7,10,13,16,19-Dokosapentaensäure, 4,7,10,13,16,19-Dokosahexaensäure, 6,9,12,15,18,21-Tetrakoshexaensäure, 9,12-Oktadekadiensäure, 6,9,12-Oktadekatriensäure, 11,14-Eikosikatetraensäure, 11,14-Eikosadiensäure, 8,11,14-Eikosatriensäure, 5,8,11,14-Eikosikatetraensäure, 13,16-Doksadiensäure, 7,10,13,16-Dokosikatetraensäure, 4,7,10,13,16-Dokosapentaensäure, 9-Trans-Hexadekensäure, 9-Trans-Oktadekensäure, 8-Eikosaensäure, 5-Eikosaensäure, Plasmalogen-Fettsäuren, 5b-Cholestan-3b-ol, 5a-Cholestan-3b-ol, 5-Cholesten-3b-ol, 5,24-Cholestadien-3b-ol, 5-Cholestan-25a-methyl-3b-ol, 5-Cholesten-24b-ethyl-3b-ol und 5,22-Cholestadien-24b-ethyl-3b-ol aufweist wobei jede dieser Verbindungen als Zusammensetzung oder Komponente eines Lipidmoleküls auftritt.

6. Verfahren nach Anspruch 1 oder 2,
**dadurch gekennzeichnet,**
**dass** die Lipidstoffwechselprodukte aus der Gruppe ausgewählt sind, die die Fettsäure 16:0, 18:0, 16:1 n7, 18:1n7, 18:1n9, 18:3n3, 20:5n3, 22:5n3, 22:6n3, 18:2n6, 18;3n6, 20:3n6 und 20:4n6 aufweist, wobei jede dieser Fettsäuren als Zusammensetzung oder Komponente eines Lipidmoleküls auftritt.

7. Verfahren nach Anspruch 1 oder 2,
**dadurch gekennzeichnet,**
**dass** die Lipidstoffwechsefprodukte aus der Gruppe ausgewählt sind, die 5b-Cholestan-3b-ol, 5a-Cholestan-3b-ol, 5-Cholesten-3b-ol, 5,24-Cholestadien-3b-ol, 5-Cholestan-25a-methyl-3b-ol, 5-Cholestan-24b-methyl-3b-ol, 5-Cholesten-24b-ethyl-3b-ol und 5,22-Cholestadien-24b-ethyl-3b-ol aufweist, wobei jede dieser Verbindungen als Zusammensetzung oder Komponente eines Lipidmoleküls auftritt.

8. Verfahren nach Anspruch 1 oder 2,
**dadurch gekennzeichnet,**
**dass** der innere Standard aus der Gruppe ausgewählt ist, die Diheptadekanol-Phosphatidylcholin, Dipentadekaenoyl-Phosphatidylethanolamin, Tetraheptadekenoyl-Kardiolipin, Diheptadekenoyl-Phosphatidylserin, Pentadekenoyl-Sphingomyelin, Heptadekanoyl-Lysophosphatidylcholin, Triheptadekaenoyl-Glycerid, Pentadekaensäure, Heptadekan-Cholesterol-Ester und freies Fukosterol umfasst.

9. Verfahren nach Anspruch 1 oder 2,
**dadurch gekennzeichnet,**
**dass** der innere Standard Heptadekan-1-heptadekanoyl-2-lyso-phosphatidylcholin für die Lipidklasse der Lysophospholipide, N-Pentadekenoyl-D-erythrosphingosylphorylcholin für die Lipidklasse der Sphingomyeline, 1,2-Diheptadekanoylphosphatidylcholin für die Lipidklasse der Phosphatidylcholine, 1,2-Diheptadekenoylphosphatidylethanolamin für die Klasse der Phosphatidyl ethanolamine, 1,2-Diheptadekenoylphosphatidylserin für die Lipidklasse der Phosphatidylserine, Pentadekaensäure für die Lipidklasse der freien Fettsäuren, Triheptadekansäure für die Lipidklasse der Triacylglyceride, 1,1',2,2'-Tetraheptadekaenoyl-kardiolipin für die Lipidklasse der Kardiolipine, Cholesteryl-heptadekanoat für die Lipidklasse der Cholesterolester und Stigmasterol für die Lipidklasse der freien Sterola umfasst.

10. Verfahren nach einem der vorhergehenden Ansprüche,
**dadurch gekennzeichnet,**
**dass** das Trennen die Chromatografie und/oder das Messen die Chromatografie umfasst.

11. Verfahren nach Anspruch 1 oder 2,
**dadurch gekennzeichnet,**
**dass** das Anzeigen mittels einer Webseite zur Ansicht erfolgt.

12. Verfahren zur Bestimmung einer den Stoffwechsel betreffenden Wirkung eines Zustands,
**dadurch gekennzeichnet,**
**dass** es folgende Schritte umfasst:
- eine Vielzahl von quantitativen Lipoprofilen gemäß Anspruch 1 wird analysiert, wobei die Vielzahl der quantitativen Lipoprofile ein quantitatives Lipoprofil von einem Objekt, das dem Zustand unterworfen worden ist, und ein quantitatives Kontroll-Lipoprofil aufweist und
- Schlüsse über die den Stoffwechsel betreffende Wirkung des Zustands werden gezogen, wobei diese Schiüsse auf Unterschiede oder Ähnlichkeiten zwischen den quantitativen Lipoprofilen des Objekts und den quantitativen Kontroll-Lipoprofifen basieren.

13. Verfahren nach Anspruch 12,
**dadurch gekennzeichnet,**
**dass** der Zustand Folgendes umfasst;
- einen Genotyp,
- einen nicht menschlichen, genetischen Ausfall,
- eine Nahrungsmittelbeschränkung oder -ergänzung,
- eine Krankheit oder einen Krankheitszustand,
- eine Anwendung eines Toxins oder vermuteten Toxins oder
- eine Anwendung eines pharmazeutischen Mittels oder Anwärtermittels.

14. Verfahren nach Anspruch 12,
**dadurch gekennzeichnet,**
**dass** das Kontroll-Lipoprofil ein zusammengestelltes Lipoprofil, das aus einer Vielzahl von individuellen Lipoprofilen besteht, oder ein Vorzustands-Lipoprofil des Objekts ist.

15. Verfahren nach Anspruch 12,
**dadurch gekennzeichnet,**
**dass** der Zustand ein Hormon oder eine Hormonbehandlung umfasst oder eine Fettleibigkeit oder eine Diabetes beeinflusst.

16. Verfahren nach einem der Ansprüche 1 bis 15,
**dadurch gekennzeichnet,**
**dass** ein gedruckter Bericht erstellt wird.

17. Verfahren nach Anspruch 4,
**dadurch gekennzeichnet,**
**dass** die Datenbank Folgendes aufweist:
(1) eine Profiltabelle mit einem quantitativen Lipidstoffwechselprodukt-Profil eines biologischen Musters eines Individuums, das einen Zustand auf weist, wobei das quantitative Lipidstoffwechselprodukt-Profil eine mengenmäßige Messung eines Lipidstoffwechselprodukt-Profils umfasst und die mengenmäßige Messung **dadurch** erfolgt, dass ein innerer Standard für das Lipidstoffivechselproduktt verwendet wird, so dass die mengenmäßige Messung in eine Datenbank aufnehmbar ist,
(2) eine Musterinformationstabelle mit einer Musteraufzeichnung für das quantitative Lipidstoffwechselprodukt-Profil,
(3) eine Zustandsinformationstabelle miteiner Zustandsaufzeichnung für das quantitative Lipidstoffwechselprodukt-Profil und
(4) eine Filterinformationstabelle mit einem Filter des quantitativen Lipidstoff wechselprodukt-Proflls für einen gewünschten Zustand.

18. Verfahren nach Anspruch 4,
**dadurch gekennzeichnet,**
**dass** es ferner eine Benutzer-Anpassungsschaltung umfasst, die mit einem ProzessQr arbeitet, der mit der Datenbank zusammenwirkt, wobei diese Benutzer-Anpassungsschaltung Folgendes umfasst:
- Mittel, die Einstellungen für die Auswahl eines Mustersatzes vorsehen,
- Mittel, die Einstellungen für die Auswahl eines Zustandssatzes vorsehen,
- Mittel, die Einstellungen für die Auswahl eines Lipidstoffwechselprodukt-Satzes vorsehen, und
- mittel, die die quantitativen Lipidstoffwechselprodukt-Profile anzeigen, die den ausgewählten Mustern und Zuständen entsprechen, wobei jedes angezeigte, quantitative Lipidstoffwechselprodukt-Profil aus mengenmäßigen Messungen des ausgewählten Lipidstoffwechselprodukts stammt.

19. Verfahren nach Anspruch 18,
**dadurch gekennzeichnet,**
**dass** die Benutzer-Anpassungsschaltung ferner einen Anzeigebereich umfasst, der den Wert einer mengenmäßigen Messung eines Lipidstoffwechselprodukts innerhalb der quantitativen Lipidstoffwechselprodukt-Profile der ausgewählten Muster und Zustände anzeigt.

20. Verfahren nach Anspruch 18,
**dadurch gekennzeichnet,**
**dass** die Benutzer-Anpassungsschaltung ferner Folgendes umfasst:
- Mittel zum Vergleich der quantitativen Lipidstoffwechselprodukt-Profile, die einem ersten Satz aus ausgewählten Mustern und Zuständen entsprechen, mit den quantitativen Lipidstoffwechselprodukt-Profilen, die einem zweiten Satz aus ausgewählten Mustern und Zuständen entsprechen, und
- Mittel zur Anzeige des Vergleichs.

21. Verfahren nach Anspruch 18,
**dadurch gekennzeichnet,**
**dass** die Benutzer-Anpassungsschaltung Folgendes umfasst:
- für eine Vielzahl von Lipidstoffwechselprodukten eine Darstellung einer beobachteten Menge von mindestens einem Lipidstoffwechselprodukt für ein zweites biologisches Muster, wobei die Darstellung geeignet ist, einen Benutzerhinweis zu-verarbeiten, dass eine weitere Information In Bezug auf ein ausgewähltes Lipidstoffwechselprodukt gewünscht wird,

22. Verfahren nach Anspruch 1 oder 2,
**dadurch gekennzeichnet,**
**dass** jede der mengenmäßigen Messungen der Vielzahl der Lipidstoffwechselprodukte in den Fraktionen, die als quantitative Lipidstoffwechsetprodukt-Profile gespeichert sind, als Massen- oder Konzentrationsmessung gespeichert wird.

23. Verfahren nach Anspruch 1 oder 2,
**dadurch gekennzeichnet,**
**dass** die Mengen von mehr als 35 individuellen Fettsäuren jeder der Lipidklassen gemessen werden.

24. Verfahren nach Anspruch 1 oder 2,
**dadurch gekennzeichnet,**
**dass** die Vielzahl der Lipidklassen eine Lipidklasse umfasst, die aus der Gruppe ausgewählt ist, die Phosphatidylcholine. Phosphatidylserine, Phosphatidylinositole, Phosphatidylethanolamine und Kardiolipin enthält.

25. Verfahren nach Anspruch 1 oder 2,
**dadurch gekennzeichnet,**
**dass** eine Zunahme oder Abnahme eines gemessenen Lipidstoffiuechselpradukts bei der Anzeige der Laufkarte durch die Farbe und der relevante Betrag der Zunahme oder Abnahme durch die Intensität dieser Farbe angezeigt wird.

26. Verfahren nach Anspruch 1 oder 2.
**dadurch gekennzeichnet,**
**dass** die Vielzahl der Lipidklassen jeweils Phospholipide, Glyceride und andere Lipide aufweist.

## Revendications

1. Méthode pour analyser une pluralité de profils métaboliques lipidiques quantitatifs, connus également sous le nom de profils lipomiques, comprenant les étapes consistant à :
(a) engendrer un profil métabolique lipidique quantitatif, comprenant :
(i) la séparation d'un échantillon biologique en fractions sur la base d'une pluralité de catégories de lipides, au moins un étalon interne quantitatif étant incorporé pour chaque catégorie de lipides ; et
(ii) la mesure de la quantité d'une pluralité de métabolites lipidiques, dans chacune des fractions, les mesures quantitatives de la pluralité de métabolites lipidiques dans les fractions étant mémorisées comme profil métabolique lipidique quantitatif;
(b) désigner une pluralité de ces profils métaboliques lipidiques quantitatifs;
(c) identifier une pluralité de différences ou de similarités dans les métabolites lipidiques mesurés entre la pluralité de profils métaboliques lipidiques quantitatifs désignés ; et
(d) représenter la pluralité de différences ou de similarités dans les métabolites lipidiques entre la pluralité de profils métaboliques lipidiques quantitatifs, la représentation comprenant la représentation d'une carte thermique, de manière à permettre à l'utilisateur une comparaison aisée entre la pluralité de profils métaboliques lipidiques.

2. Méthode suivant la revendication 1, qui comprend en outre l'étape consistant à
engendrer chacun de la pluralité de profils métaboliques lipidiques quantitatifs désignés en (b), la production de chacun des profils métaboliques lipidiques quantitatifs comprenant :
(i) la séparation d'un échantillon biologique en fractions correspondant chacune à l'une d'une pluralité de catégories de lipides, au moins un étalon interne quantitatif étant incorporé pour chaque catégorie de lipides ; et
(ii) la mesure de la quantité d'une pluralité de métabolites lipidiques dans chacune des fractions, les mesures quantitatives de la pluralité de métabolites lipidiques dans les fractions étant mémorisées comme profil métabolique lipidique quantitatif.

3. Méthode suivant la revendication 1 ou 2, dans laquelle la pluralité de catégories de lipides est constituée de catégories qui comprennent des lyso-phosphatidylcholines, des sphingomyélines, des phosphatidylcholines, des phosphatidylsérines, des phosphatidylinositols, des phosphatidyléthanolamines, des cardiolipines, des acides gras libres, des monoacylglycérides, des diacylglycérides, des triacylglycérides et/ou des esters de cholestérol.

4. Méthode suivant la revendication 1 ou 2, qui comprend en outre l'assemblage de la pluralité de profils lipomiques dans une base de données.

5. Méthode suivant la revendication 1 ou 2, dans laquelle les métabolites lipidiques sont choisis dans le groupe consistant en l'acide tétradécanoïque, l'acide pentadécanoïque, l'acide hexadécanoïque, l'acide heptadécanoïque, l'acide octadécanoïque, l'acide eicosanoïque, l'acide docosanoïque, l'acide tétracosanoïque, l'acide 9-tétradécénoïque, l'acide 9-hexadécénoïque, l'acide 11-octadécénoïque, l'acide 9-octadécénoïque, l'acide 11-eicosénoïque, l'acide 5,8,11-eicosatriénoïque, l'acide 13-docosénoïque, l'acide 15-tétracosénoïque, l'acide 9,12,15-octadécatriénoïque, l'acide 6,9,12,15-octadécatétraénoïque, l'acide 11,14,17-eicosatriénoïque, l'acide 8,11,14,17-eicosatétraénoïque, l'acide 5,8,11,14,17-eicosapentaénoïque, l'acide 7,10,13,16,19-docosapentaénoïque, l'acide 4,7,10,13,16,19-docosahexaénoïque, l'acide 6,9,12,15,18,21-tétracosahexaénoïque, l'acide 9,12-octadécadiénoïque, l'acide 6,9,12-octadécatriénoïque, l'acide 11,14-eicosadiénoïque, l'acide 8,11,14-eicosatriénoïque, l'acide 5,8,11,14-eicosatétraénoïque, l'acide 13,1-6-docosadiénoïque, l'acide 7,10,13,16-docosatétraénoïque, l'acide 4,7,10,13,16-docosapentaénoïque, l'acide 9-trans-hexadécénoïque, l'acide 9-trans-octadécénoïque, l'acide 8-eicosaénoïque, l'acide 5-eicosaénoïque, des acides gras plasmalogènes, le 5b-cholestane-3b-ol, le 5a-cholestane-3b-ol, le 5-cholestène-3b-ol, le 5,24-cholestadiène-3b-ol, le 5-cholestane-25a-méthyl-3b-ol, le 5-cholestane-24b-méthyl-3b-ol, le 5-cholestène-24b-éthyl-3b-ol et le 5,22-cholestadiène-24b-éthyl-3b-ol, chacun sous forme d'un composé ou d'un constituant d'une molécule de lipide.

6. Méthode suivant la revendication 1 ou 2, dans laquelle les métabolites lipidiques sont choisis dans le groupe consistant en les acides gras 16:0, 18:0, 16:1n7 ; 18:1n7 ; 18:1n9 ; 18:3n3 ; 20:5n3 ; 22:5n3 ; 22:6n3 ; 18:2n6 ; 18:3n6 ; 20:3n6 ; et 20:4n6, chacun sous forme d'un composé ou d'un constituant d'une molécule de lipide.

7. Méthode suivant la revendication 1 ou 2, dans laquelle les métabolites lipidiques sont choisis dans le groupe consistant en 5b-cholestane-3b-ol, 5a-cholestane-3b-ol, 5-cholestène-3b-ol, 5,24-cholestadiène-3b-ol, 5-cholestane-25a-méthyl-3b-ol, 5-cholestane-24b-méthyl-3b-ol, 5-cholestène-24b-éthyl-3b-ol et 5,22-cholestadiène-24b-éthyl-3b-ol, chacun sous forme d'un composé ou d'un constituant d'une molécule de lipide.

8. Méthode suivant la revendication 1 ou 2, dans laquelle l'étalon interne est choisi dans le groupe consistant en la diheptadécanoyl-phosphatidylcholine, la dipentadécanaénoyl-phosphatidyléthanolamine, la tétraheptadécénoylcardiolipine, la diheptadécénoyl-phosphatidylsérine, la pentadécénoyl-sphingomyéline, l'heptadécanoyl-lysophosphatidylcholine, le triglycéride triheptadécaénoylique, l'acide pentadécaénoïque, l'ester heptadécanoïque de cholestérol et le fucostérol libre.

9. Méthode suivant la revendication 1 ou 2, dans laquelle l'étalon interne est la 1-heptadécanoyl-2-lyso-phosphatidylcholine heptadécanoïque pour la catégorie de lipides consistant en lysophospholipides, la N-pentadécénoyl-D-érythrosphingosylphorylcholine pour la catégorie de lipides consistant en sphingomyélines, la 1,2-diheptadécanoylphosphatidylcholine pour la catégorie de lipides consistant en phosphatidylcholines, la 1,2-diheptadécanoylphosphatidyléthanolamine pour la catégorie de lipides consistant en phosphatidyléthanolamines, la 1,2-diheptadécénoylphosphatidylsérine pour la catégorie de lipides consistant en phosphatidylsérines, l'acide pentadécaénoïque pour la catégorie de lipides consistant en acides gras libres, l'acide triheptadécaénoïque pour la catégorie de lipides consistant en triacylglycérides, la 1,1',2,2'-tétraheptadécaénoyl-cardiolipine pour la catégorie de lipides consistant en cardiolipines, l'heptadécanoate de cholestéryle pour la catégorie de lipides consistant en esters de cholestérol et le stigmastérol pour la catégorie de lipides consistant en stérols libres.

10. Méthode suivant l'une quelconque des revendications précédentes, dans laquelle la séparation comprend la chromatographie et/ou la mesure comprend la chromatographie.

11. Méthode suivant la revendication 1 ou 2, dans laquelle la représentation engendre une page web pour examen.

12. Méthode pour la détermination d'un effet métabolique d'une condition, comprenant les étapes consistant
à analyser une pluralité de profils lipomiques quantitatifs suivant la revendication 1, la pluralité de profils lipomiques quantitatifs comprenant un profil lipomique quantitatif d'un sujet qui a été soumis à la condition et un profil lipomique témoin quantitatif ; et
tirer les conclusions concernant l'effet métabolique de la condition d'après les différences ou similarités entre le profil lipomique quantitatif du sujet et le profil lipomique témoin quantitatif.

13. Méthode suivant la revendication 12, dans laquelle la condition comprend :
un génotype ;
une inactivation ("knockout") génétique non humaine ;
une limitation ou supplémentation alimentaire ;
une maladie ou un état pathologique ;
l'application d'une toxine ou toxine supposée ; ou
l'application d'un agent pharmaceutique ou agent candidat.

14. Méthode suivant la revendication 12, dans laquelle le profil lipomique témoin est :
un profil lipomique compilé assemblé à partir d'une pluralité de profils lipomiques individuels ; ou
un profil lipomique, avant condition, provenant du sujet.

15. Méthode suivant la revendication 12, dans laquelle la condition comprend une hormone ou un traitement hormonal ou a une influence sur l'obésité ou le diabète.

16. Méthode suivant l'une quelconque des revendications 1 à 16, comprenant en outre la production d'un rapport imprimé.

17. Méthode suivant la revendication 4, dans laquelle la base de données comprend :
(1) une table de profils comprenant un profil métabolique lipidique quantitatif à partir d'un échantillon biologique provenant d'un individu présentant une condition, le profil métabolique lipidique quantitatif comprenant une mesure quantifiée d'un métabolite lipidique et la mesure quantifiée étant obtenue en utilisant un étalon interne pour le métabolite lipidique de sorte que la mesure quantifiée soit apte à l'intégration dans une base de données ;
(2) une table de critères d'échantillons comprenant un enregistrement d'échantillons pour le profil métabolique lipidique quantitatif;
(3) une table de critères de conditions comprenant un enregistrement de conditions pour le profil métabolique lipidique quantitatif ; et
(4) une table de critères comprenant un filtre du profil métabolique lipidique quantitatif pour une condition désirée.

18. Méthode suivant la revendication 4, comprenant en outre une interface d'utilisateur pour le traitement opérationnel avec un processeur afin d'effectuer l'opération de la base de données, l'interface d'utilisateur comprenant :
un moyen pour fournir des réglages pour la sélection d'une série d'échantillons,
un moyen pour fournir des réglages pour la sélection d"une série de conditions,
un moyen pour fournir des réglages pour la sélection d'une série de métabolites lipidiques, et
un moyen pour représenter les profils métaboliques lipidiques quantitatifs correspondant aux échantillons et conditions choisis, chaque profil métabolique lipidique quantitatif représenté consistant en les mesures quantifiées des métabolites lipidiques choisis.

19. Méthode suivant la revendication 18, dans laquelle l'interface d'utilisateur comprend en outre une zone d'affichage qui représente la valeur d'une mesure quantifiée d'un métabolite lipidique dans lés profils métaboliques lipidiques quantitatifs des échantillons et conditions choisis.

20. Méthode suivant la revendication 18, dans laquelle l'interface d'utilisateur comprend en outre
un moyen pour comparer les profils métaboliques lipidiques quantitatifs correspondant à une première série d'échantillons et de conditions choisis avec les profils métaboliques lipidiques quantitatifs correspondant à une seconde série d'échantillons et de conditions choisis, et
un moyen pour représenter la comparaison.

21. Méthode suivant la revendication 18, dans laquelle l'interface d'utilisateur comprend :
pour une pluralité de métabolites lipidiques, une présentation d'une quantité observée d'au moins un métabolite lipidique pour un premier échantillon biologique par rapport à une quantité observée dudit au moins un métabolite lipidique pour un second échantillon biologique, la présentation étant apte à accepter une indication d'utilisateur qu'une information supplémentaire est désirée concernant le métabolite lipidique choisi.

22. Méthode suivant la revendication 1 ou 2, dans laquelle chacune des mesures quantitatives de la pluralité de métabolites lipidiques dans les fractions mémorisées sous forme du profil métabolique quantitatif est mémorisée sous forme d'une mesure de masse ou de concentration.

23. Méthode suivant la revendication 1 ou 2, dans laquelle les quantités supérieures à 35 acides gras individuels à partir de chacune des catégories de lipides sont mesurées.

24. Méthode suivant, la revendication 1 ou 2, dans laquelle la pluralité de catégories de lipides comprend une catégorie de lipides choisie dans le groupe consistant en les phosphatidylcholines, les phosphatidylsérines, les phosphatidylinositols, les phosphatidyléthanolamines et la cardiolipine.

25. Méthode suivant la revendication 1 ou 2, dans laquelle une augmentation ou diminution d'un métabolite lipidique mesuré est indiquée sur la représentation de cartes thermiques par la couleur, et la quantité intéressante d'augmentation ou de diminution est indiquée par l'intensité de cette couleur.

26. Méthode suivant la revendication 1 ou 2, dans laquelle la pluralité de catégories de lipides comprend chacune des catégories de phospholipides, de glycérides et d'autres lipides.
